(19) Europäisches Patentamt
European Patent Office
Office européen des brevets

(11) EP 4 079 866 A1

(12) # EUROPEAN PATENT APPLICATION

(43) Date of publication:
**26.10.2022 Bulletin 2022/43**

(21) Application number: **22169313.8**

(22) Date of filing: **21.04.2022**

(51) International Patent Classification (IPC):
***C12Q 1/6895*** *(2018.01)*

(52) Cooperative Patent Classification (CPC):
**C12Q 1/6895**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.04.2021 US 202163177640 P**
**21.04.2021 US 202163177664 P**
**21.04.2021 US 202163177676 P**

(71) Applicant: **Plant Sciences Inc.**
**Watsonville, CA 95076 (US)**

(72) Inventors:
- **JACKSON, Eric**
**WATSONVILLE, CA 95076 (US)**
- **D. NELSON, Michael**
**WATSONVILLE, CA 95076 (US)**

(74) Representative: **Regimbeau**
**20, rue de Chazelles**
**75847 Paris Cedex 17 (FR)**

(54) **MOLECULAR MARKERS FOR IDENTIFICATION OF FUSARIUM RESISTANCE IN STRAWBERRY PLANTS AND USES THEREOF**

(57) The invention relates to single nucleotide polymorphism (SNP) markers that provide improved resistance to one or more diseases and/or improved agronomic traits, such as improved resistance to *Fusarium* infection, improved resistance to *Macrophomina* infection, improved resistance to *Colletotrichum resistance,* improved remontancy and/or improved berry weight. Also described are compositions and methods of using the SNP markers in selecting, obtaining, or developing strawberry plants or varieties with the given improved traits.

EP 4 079 866 A1

SEQUENCE LISTING

<110> Plant Sciences Inc.

<120> MOLECULAR MARKERS FOR IDENTIFICATION OF FUSARIUM RESISTANCE IN STRAWBERRY PLANTS AND USES THEREOF

<130> B77598 D42648

<150> US63/177640
<151> 2021-04-21

<150> US63/177664
<151> 2021-04-21

<150> US63/177676
<151> 2021-04-21

<160> 102

<170> PatentIn version 3.5

<210> 1
<211> 14
<212> DNA
<213> Fragaria sp.

<400> 1
tgattgtctg agaa 14

<210> 2
<211> 15
<212> DNA
<213> Fragaria sp.

<400> 2
tgaagtatga attaa 15

<210> 3
<211> 12
<212> DNA
<213> Fragaria sp.

<400> 3
aagggccatg tg 12

<210> 4
<211> 12
<212> DNA
<213> Fragaria sp.

<400> 4
caccgcctgg tt 12

<210> 5
<211> 12
<212> DNA
<213> Fragaria sp.

<400> 5
aaccatccac aa 12

<210> 6
<211> 11
<212> DNA
<213> Fragaria sp.

<400> 6
tggcctccga g 11

<210> 7
<211> 12
<212> DNA
<213> Fragaria sp.

<400> 7
cagctttgga at 12

<210> 8
<211> 14
<212> DNA
<213> Fragaria sp.

<400> 8
atgcataaga tgtg 14

<210> 9
<211> 12
<212> DNA
<213> Fragaria sp.

<400> 9
actgtaacga ca 12

<210> 10
<211> 15
<212> DNA
<213> Fragaria sp.

<400> 10
agatagctaa actca 15

<210> 11
<211> 12
<212> DNA
<213> Fragaria sp.

<400> 11
ggatckgtga ag 12

<210> 12
<211> 12
<212> DNA
<213> Fragaria sp.

<400> 12
ctcgccgttc ta 12

<210> 13
<211> 12
<212> DNA
<213> Fragaria sp.

<400> 13
gatcyacatt ct 12

<210> 14
<211> 12
<212> DNA
<213> Fragaria sp.

<400> 14
atccttatcr ac 12

<210> 15
<211> 12
<212> DNA
<213> Fragaria sp.

<400> 15
tggtggttgt tc 12

<210> 16
<211> 12
<212> DNA
<213> Fragaria sp.

<400> 16
gccacgaaat ga 12

<210> 17
<211> 12
<212> DNA
<213> Fragaria sp.

<400> 17
aagcatcatc aa 12

<210> 18
<211> 401
<212> DNA
<213> Fragaria sp.

<400> 18
tgttgatgat gatgaaagaa gaatgagagc aacaagagtg aagagaaagc acttgtgcct 60

ccaagaacct aacattgttg aatagtaata cactttggaa ttgaaaagaa gtgatatata 120

ctcaatagtt ttgtactctc aacttactga gaaggcagca gacaagttga gtaatgtaag 180

gagactgcca acttgattgt ktgagaaaag atgtggcaat ggcagacagt gaggtatttg 240

ataattctta aactgtkaag gctaagcagc tcaggctgtt gctctcattg tatagaatct 300

gtgtaagtga aagagaaaag aatttagtgg attttattgt tcttaattgg aacccctttt 360

ttgagttggg tataagaatt aaattgtttc tatgtctggt a 401

<210> 19
<211> 20
<212> DNA
<213> Artificial Sequence

<220>
<223> FUS1 Forward Primer

<400> 19
cttactgaga aggcagcaga 20

<210> 20
<211> 18
<212> DNA
<213> Artificial Sequence

<220>
<223> FUS1 Reverse Primer

<400> 20
agctgcttag ccttgaca 18

<210> 21
<211> 14
<212> DNA
<213> Artificial Sequence

<220>
<223> FUS1 T allele probe

<220>
<221> misc_feature
<222> (1)..(1)

<220>
<221> misc_feature
<222> (14)..(14)

<400> 21
ttctcaaaca atca 14

<210> 22
<211> 12
<212> DNA
<213> Artificial Sequence

<220>
<223> FUS1 C allele probe

<220>
<221> misc_feature

<222> (1)..(1)

<220>
<221> misc_feature
<222> (12)..(12)

<400> 22
ctcagacaat ca 12

<210> 23
<211> 400
<212> DNA
<213> Fragaria sp.

<400> 23
attgcttgac caaaatacaa aatgcaaata ccttggtaga gtaaggagga agctttaggt 60

aattagcgca tgtcattaca ctaggtagat catcatctgc caattctgaa gccccagtcc 120

cattaggtgc tgcattattc gcagttgaag aatgctgcag cagacaagga aacatcaaca 180

catcaataca ccttgaagta yaattaacca ttacaagttt gagggaagca agcagccaaa 240

aatgaggcat gaaaagaagc catagaatct cgctgtacct ttctcacaat ggtgagcttt 300

ggattgagca ctgccaaacc accaggggga agcctaggcg caccagtaac aaactggcaa 360

aaggcacgct gctgctcggg cgtgaactca cccatgatct 400

<210> 24
<211> 21
<212> DNA
<213> Artificial Sequence

<220>
<223> FUS2 Forward Primer

<400> 24
cccattaggt gctgcattat t 21

<210> 25
<211> 21
<212> DNA
<213> Artificial Sequence

<220>
<223> FUS2 Reverse Primer

<400> 25
aggtacagcg agattctatg g 21

<210> 26
<211> 14
<212> DNA
<213> Artificial Sequence

<220>
<223> FUS2 C allele probe

<220>
<221> misc_feature
<222> (1)..(1)

<220>
<221> misc_feature
<222> (14)..(14)

<400> 26
tgaagtacga atta 14

<210> 27
<211> 15
<212> DNA
<213> Artificial Sequence

<220>
<223> FUS2 T allele probe

<220>
<221> misc_feature
<222> (1)..(1)

<220>
<221> misc_feature
<222> (15)..(15)

<400> 27
tgaagtatga attaa 15

<210> 28
<211> 401
<212> DNA
<213> Fragaria sp.

<400> 28
ttctaaattt ctggcgacgc atgtaatcca tgtgaattaa taagttgtgc gtagcttgat 60

tatactgcaa atggaattgg ggtttgagct tacacattct cgaacggcag aaatgagagg 120

agctagaaag cttaagagag cagcagcggc aatattagga agacttggga gatcgagtga 180

ggagggaagg aagagaaggg mcatgtgaaa aaaggcagcc tttcttgttt tgatgtttgt 240

aaagcgacca cgccccgttt ggctgcaatc atgtggtcta ctttgttata attccattcc 300

ccttcactac tctctaattc ctcccatttc ttttttgtc gtcacccttt gtccccttc 360

cctaagtacc ttgttcctct acaacattat gaaaatcaca t 401

<210> 29
<211> 21
<212> DNA
<213> Artificial Sequence

<220>
<223> FUS3 Forward Primer

<400> 29
cagcggcaat attaggaaga c 21

<210> 30
<211> 19
<212> DNA
<213> Artificial Sequence

<220>
<223> FUS3 Reverse Primer

<400> 30
cgtggtcgct ttacaaaca 19

<210> 31
<211> 10
<212> DNA
<213> Artificial Sequence

<220>
<223> FUS3 C allele probe

<220>
<221> misc_feature
<222> (1)..(1)

<220>
<221> misc_feature
<222> (10)..(10)

<400> 31
agggccatgt 10

<210> 32
<211> 11
<212> DNA
<213> Artificial Sequence

<220>
<223> FUS3 A allele probe

<220>
<221> misc_feature
<222> (1)..(1)

<220>
<221> misc_feature
<222> (11)..(11)

<400> 32
agggacatgt g 11

<210> 33
<211> 401
<212> DNA
<213> Fragaria sp.

<400> 33
gaacaacaac aagatgcaac caagaaaaac agcttagttt tgttgggcat cagttaagtc 60

cttgtccttc tgcagccttg tacagctctt cggtacatgc atagtgtgaa agtgtggctc 120

ctttggaatg gttgcaggcc ttcttccccg tgatgatgac cgagaaggag ttcgaacctt 180

ggttgctgaa gaaacaccgc mtggttttgc tgctttcttg ggtcttatat ctgttgatat 240

gctttcgttt attacactga gttgacaagg agtcttggac gaaaccacag acttcttttt 300

acttgaaaca accctgcaca agaaaagttt agtaagacca agtttcaaca agagcacacc 360

atgtatttat catgatggaa aattttagca agaccaagtt t 401

<210> 34
<211> 20
<212> DNA
<213> Artificial Sequence

<220>
<223> FUS4 Forward Primer

<400> 34
gtgatgatga ccgagaagga 20

<210> 35
<211> 20
<212> DNA
<213> Artificial Sequence

<220>
<223> FUS4 Reverse Primer

<400> 35
ttcttgtgca gggttgtttc 20

<210> 36
<211> 10
<212> DNA
<213> Artificial Sequence

<220>
<223> FUS4 A allele probe

<220>
<221> misc_feature
<222> (1)..(1)

<220>
<221> misc_feature
<222> (10)..(10)

<400> 36
accgcatggt 10

<210> 37
<211> 10

<212> DNA
<213> Artificial Sequence

<220>
<223> FUS4 C allele probe

<220>
<221> misc_feature
<222> (1)..(1)

<220>
<221> misc_feature
<222> (10)..(10)

<400> 37
accgcctggt 10

<210> 38
<211> 401
<212> DNA
<213> Fragaria sp.

<400> 38
ccattgcggc attgccacta atggaactcc taaactcaga gactccaaag ttgagttcca 60

accacaatgt gtaatgaagc acccaacagc ctcatgagcc aaaacctcta gttgggagca 120

ccatgaaacc accaaaccct tctcagctgt ctcctcgatg aacccttcgg ggactttagc 180

tgcttctgat tctctaacca yccacaagaa tttgcttttg ctcctcttca aaccccacgc 240

cagttcctcc atttgctcaa gtccaagttg tgcagcactg ccgaatgaga tgtaagcaac 300

agagttcttc ggatgtttgt ttaaccattt catgcatgca tcattgctgg atttaaagag 360

gtcaacgcca taacctttgt cgtctttaag tcggttatcc a 401

<210> 39
<211> 22
<212> DNA
<213> Artificial Sequence

<220>
<223> FUS5 Forward Primer

<400> 39
ggactttagc tgcttctgat tc 22

<210> 40
<211> 20
<212> DNA
<213> Artificial Sequence

<220>
<223> FUS5 Reverse Primer

<400> 40
ctgcacaact tggacttgag 20

<210> 41
<211> 11
<212> DNA
<213> Artificial Sequence

<220>
<223> FUS5 C allele probe

<220>
<221> misc_feature
<222> (1)..(1)

<220>
<221> misc_feature
<222> (11)..(11)

<400> 41
accacccaca a 11

<210> 42
<211> 12
<212> DNA
<213> Artificial Sequence

<220>
<223> FUS5 T allele probe

<220>
<221> misc_feature
<222> (1)..(1)

<220>
<221> misc_feature
<222> (12)..(12)

<400> 42
aaccatccac aa 12

<210> 43
<211> 401
<212> DNA
<213> Fragaria sp.

<400> 43
gtgacggcgc tgttggtggc gctgcttcct ccgtcgtagt ggcagcgctt gtgtcctccc 60

aaggcctgcc cggtcggaaa acacttgtgg cagatggtgc actcgtggct cctcccgcta 120

acggcggtga cagccggggt ttcaaccttg acggcggagg agacggtgga gtcggacttg 180

cggtggctag ccttgtggcc kccgagagcc tggtaagacc cgaaagcctt gctgcagacg 240

acgcacttgt aagaggccgg gggatgcggt gggagttgcg cgacggtggt gtcggcggcg 300

ttggtggtgc cgcgggcgag gagcaagagg cagaaggcca tgtactcctc ctcggaaagt 360

gatgagtcct cgaaacgcgg ccgcttggag cgcttacgct t 401

<210> 44
<211> 401
<212> DNA
<213> Fragaria sp.

<400> 44
agaaacaatg taaacagcca ttggcagtgg ctgatcatca agctcccgag ccgcttctgc 60

cgccccctca gcctccctct gcttgcacac aagagtctcc caagaccggc acatggcata 120

agggccaacc cacgaccctg cagcaaggtt ataacccttg cccgcctgaa tcaagttatg 180

aattgagaac accgcagctt yggaatcacc aaacagccgc aggatcttaa catactcttg 240

ttccaatccg caagacttgt ccacaggccg cctccaagat cttcccagcc tgtggaaaag 300

caatgcctgc gccacaagca tctggctgct ccgaatcata cacccccaat tcacatcact 360

cgtaaacttg gaatctccac cctctatcaa atcgaatcct c 401

<210> 45
<211> 20
<212> DNA
<213> Artificial Sequence

<220>
<223> FUS7 Forward Primer

<400> 45
cccgcctgaa tcaagttatg 20

<210> 46
<211> 21
<212> DNA
<213> Artificial Sequence

<220>
<223> FUS7 Reverse Primer

<400> 46
gcggattgga acaagagtat g 21

<210> 47
<211> 11
<212> DNA
<213> Artificial Sequence

<220>
<223> FUS7 C allele probe

<220>
<221> misc_feature
<222> (1)..(1)

<220>
<221> misc_feature
<222> (11)..(11)

<400> 47
agcttcggaa t 11

<210> 48
<211> 11
<212> DNA
<213> Artificial Sequence

<220>
<223> FUS7 T allele

<220>
<221> misc_feature
<222> (1)..(1)

<220>
<221> misc_feature
<222> (11)..(11)

<400> 48
agctttggaa t 11

<210> 49
<211> 401
<212> DNA
<213> Fragaria sp.

<400> 49
gagttcccac ttcctggaga ggaggagcga tttaaattat tgaaactcta cttaaacaag 60

tatctttctg gtgaagacaa cacctctaaa tggggcattc tgaacaagaa gcaacaacag 120

aagataacta tgcaggattt gtccgatgag gtgctccgag aggctgccag gaagacagat 180

gggttctctg gccgygagat mgctaaactc atggctggtg ttcargcagc tgtgtacggg 240

cgcccagatt gtgtactgga ctcccaattg tttaaggaga ttgtwgatta caaagttgcg 300

gagcatcacc agaggataaa actagcagct gaaggtggtg atgggatttt tggcctagct 360

tgagtgaata gaagtaggag ttgaacgtct gtcatatccc a 401

<210> 50
<211> 20
<212> DNA
<213> Artificial Sequence

<220>
<223> COL 1 Forward Primer

<400> 50
caggaagaca gatgggttct 20

<210> 51
<211> 20
<212> DNA
<213> Artificial Sequence

<220>
<223> COL 1 Rev

<400> 51
tgctccgcaa ctttgtaatc 20

<210> 52
<211> 15
<212> DNA
<213> Artificial Sequence

<220>
<223> COL 1 A allele prob

<220>
<221> misc_feature
<222> (1)..(1)

<220>
<221> misc_feature
<222> (15)..(15)

<400> 52
agatagctaa actca 15

<210> 53
<211> 13
<212> DNA
<213> Artificial Sequence

<220>
<223> COL 1 C allele probe

<220>
<221> misc_feature
<222> (1)..(1)

<220>
<221> misc_feature
<222> (13)..(13)

<400> 53
agatcgctaa act 13

<210> 54
<211> 401
<212> DNA
<213> Fragaria sp.

<400> 54
aaagygagag aaagatgcct tcttgttctg gaaggtcagc aaaagatgac aaggcaaatg 60

ggtcatctga tcaccgggca aaccakgtyg attcgacctc attacaatcg garcaattag 120

ctggagctgc taaccattct gctgcactag cagctgaraa actgaaccat gaagtggtcg 180

agtggccrag aatttacttg rstttgtcaa ggaaggagaa agaagaygat ttccttgcaa 240

tgaaaggcac gaagctgcct cagagaccca agaagagggc caagaacgtt gacagaaccc 300

ttcaggtaga aatattcatc acttattctt ggttcatcaa tttgattttg gtttttgggt 360

aattgagaaa ttggaacatg aattttaatt ttttgtgggg g 401

<210> 55
<211> 12
<212> DNA
<213> Fragaria sp.

<400> 55
acttgrsttt gt 12

<210> 56
<211> 401
<212> DNA
<213> Fragaria sp.

<400> 56
ggcccaccag cgcagaggct gcccggagca gcagcagcag ccgctcttgg ccgtcaaagt 60

cgaactcgag cagctcataa tctcgattct cgatgayccc agtgttagtc gggtcatgcg 120

ggaggccagt ttctctagtc cggcagttaa agcraccatc gaacagtctc ttaactcttc 180

atcmgcggcg gcggcagcga mctctaccgt cgccgcgaat tcgtycccaa ttggattggg 240

gttccggcck gcgggaccrc ccgccggtcg gaacatgtat ttaaatcccc ggctgcaggg 300

agcagcaggg caatcggggc aaaaccgagc agaggaagtt aaaaaagttg ctgatatttt 360

atcaaggggg aagaagagga atccggtatt ggtcggcgat t 401

<210> 57
<211> 12
<212> DNA
<213> Fragaria sp.

<400> 57
agcgamctct ac 12

<210> 58
<211> 401
<212> DNA
<213> Fragaria sp.

<400> 58
ctctttctct tcataaattc acccacctca tcaaaaagcc tcacatagta aaaatccttc 60

aaatcgatct gctcgcacaa cttcttcaac acttcaaaaa cctccttgtc attcttctcc 120

tcatgcacaa gaatctgttt tttccgcagc aatttcacat ctttctcgga ctggatcagg 180

caccccatta agtatgcata mgatgtgaya tgctgcaccg gatcatcccc aatgtgtcgc 240

tgctcgagtg caatgaggtt cttgaggagt gtttcagtgc agtgatggac taaaagaggt 300

ggcattttga acacaccatt agtaaacttg acatccaaga aactgtgtac cacggtagca 360

cattggaatt taacccctgc tctttttagc ttctttacgc a 401

<210> 59
<211> 20
<212> DNA
<213> Artificial Sequence

<220>
<223> MAC 2 Forward Primer

<400> 59
tcacatcttt ctcggactgg 20

<210> 60
<211> 22
<212> DNA
<213> Artificial Sequence

<220>
<223> MAC 2 Reverse Primer

<400> 60
cactgaaaca ctcctcaaga ac 22

<210> 61
<211> 14
<212> DNA
<213> Artificial Sequence

<220>
<223> MAC 2 A allele probe

<220>
<221> misc_feature
<222> (1)..(1)

<220>
<221> misc_feature
<222> (14)..(14)

<400> 61
cacatcttat gcat 14

<210> 62
<211> 12
<212> DNA
<213> Artificial Sequence

<220>
<223> MAC 2 C allele probe

<220>
<221> misc_feature
<222> (1)..(1)

<220>

<221> misc_feature
<222> (12)..(12)

<400> 62
acatcgtatg ca 12

<210> 63
<211> 401
<212> DNA
<213> Fragaria sp.

<220>
<221> misc_feature
<222> (218)..(220)
<223> n is a, c, g, or t

<400> 63
taaaacttcc tactctctga agccagcact aggctaagat ccgaaagata agactgcaag 60

tccctgcaat catcacaaca cacaaaaaaa aaaaagcctc acttccttgc tcacacaaca 120

gcaaacaacc aaaatcaacg gtacattatg cgttcagcag ctgaagaagc acaatgttat 180

ctctacgtat agcattactg yaacgacaca cggtcacnnn cacaaacacc acttawagca 240

gcaaactcaa agcaacraaa tcractacat ttaggaattg agctrattat tcagagtatc 300

aatcacaact gattaaaact aattcagaac aatcgaaatc gaaagattgg ttacccaatt 360

tgcaaaccag tgagggggga aatgcaccgg ccggcgtcac t 401

<210> 64
<211> 19
<212> DNA
<213> Artificial Sequence

<220>
<223> MAC 3 Forward Primer

<400> 64
tcacttcctt gctcacaca 19

<210> 65
<211> 19
<212> DNA
<213> Artificial Sequence

<220>
<223> MAC 3 Reverse Primer

<400> 65
ttgctttgag tttgctgct 19

<210> 66
<211> 11
<212> DNA
<213> Artificial Sequence

<220>
<223> MAC 3 T allele probe

<220>
<221> misc_feature
<222> (1)..(1)

<220>
<221> misc_feature
<222> (11)..(11)

<400> 66
actgtaacga c 11

<210> 67
<211> 12
<212> DNA
<213> Artificial Sequence

<220>
<223> MAC 3 C allele probe

<220>
<221> misc_feature
<222> (1)..(1)

<220>
<221> misc_feature
<222> (12)..(12)

<400> 67
actgcaacga ca 12

<210> 68
<211> 601
<212> DNA
<213> Fragaria sp.

<400> 68
gaaatttcga ctacatgtca tatatgattc agcaaaatca caaagttaaa gaattcagca 60

tccaaaattc aaacttgcaa acaaatcaac caacacaatt acatataatt cccgggaaat 120

ttgggtcctg agatgtaata cctgggataa gacccttctg aatcttctgg acctgtttgc 180

agtttacccc agaaagaacg agaaagtgag acggttggcc ttaactacct cattcagttt 240

taccgttaga gaccaaggtt aggtggactt tgaagtttgc tgcggccttc ttctgggatc 300

kgtgaagctg aacaagtctg tctgattggt caaaactgat tggtgctaca gcaacagcta 360

aaacagtgaa actcaaacag ctgccgtact cagaatgttg tgttgtgcgg tgtacgctgt 420

cactttggac cttcaataat tgagtgtact aatctacaaa ttttgtaacg ctccctgatc 480

ttaataactg tggtttaaat ataccagatc atctgcagtt cttttgcatg tgatttggtg 540

cataaagccg gtggttaagc aattgaacgc aggggatttc aatagtttat gaaattaatg 600

g 601

<210> 69
<211> 18
<212> DNA
<213> Artificial Sequence

<220>
<223> ROM 1 Forward Primer

<400> 69
taggtggact ttgaagtt 18

<210> 70
<211> 19
<212> DNA
<213> Artificial Sequence

<220>
<223> ROM 1 Reverse Primer

<400> 70
caccaatcag ttttgacca 19

<210> 71
<211> 14
<212> DNA
<213> Artificial Sequence

<220>
<223> ROM 1 G allele probe

<220>
<221> misc_feature
<222> (1)..(1)

<220>
<221> misc_feature
<222> (14)..(14)

<400> 71
gggatcggtg aagc 14

<210> 72
<211> 14
<212> DNA
<213> Artificial Sequence

<220>
<223> ROM 1 T allele probe

<220>
<221> 1misc_feature
<222> (1)..(1)

<220>
<221> 1misc_feature

<222> (14)..(14)

<400> 72
gggatctgtg aagc 14

<210> 73
<211> 401
<212> DNA
<213> Fragaria sp.

<400> 73
acaggagtgc atgtggcgct gaagtttgtt ataagctcsa gccagatcct cggcctcttg 60

acaaacgctg ccggtgaagt caaaacacat gtygcwccgg agacaatggt caacaacaag 120

aacatgagrc cgcaatcatg gtactgaggc agccacgrca cgatcacact gttcgggtga 180

aggtcgtagg atttcctcgc mgttctaacg ttgtgaactg ccgagccggm ggtgacaagy 240

accggctttg gaatcccggt agtacctgas gtgtactgaa tcaaatatar ctcgtcagct 300

ttgcagccct cgtaggacga agggctgaac tgtaaattcg agtttctagc tccggttttg 360

atatcatcgg tggatatcca gtggagattc tccaacaagt g 401

<210> 74
<211> 20
<212> DNA
<213> Artificial Sequence

<220>
<223> BW 1 Forward Primer

<400> 74
gggtgaaggt cgtaggattt 20

<210> 75
<211> 20
<212> DNA
<213> Artificial Sequence

<220>
<223> BW 1 Reverse Primer

<400> 75
ggtactaccg ggattccaaa 20

<210> 76
<211> 11
<212> DNA
<213> Artificial Sequence

<220>
<223> BW 1 A allele probe

<220>
<221> misc_feature
<222> (1)..(1)

<220>
<221> misc_feature
<222> (11)..(11)

<400> 76
tcgcagtcct a 11

<210> 77
<211> 11
<212> DNA
<213> Artificial Sequence

<220>
<223> BW 1 C allele probe

<220>
<221> misc_feature
<222> (1)..(1)

<220>
<221> misc_feature
<222> (11)..(11)

<400> 77
tcgccgtcct a 11

<210> 78
<211> 401
<212> DNA
<213> Fragaria sp.

<400> 78
tgaactcttc tgagaacaat cctgccaact gctgccatag ctcaactttt gaaggctatc 60

gaaaatatac atccgtcctt tcggtatatt tgagctgcta gccaattcct ttmgagattc 120

tctcrgtcaa atccgaaaga agagcatact tgttrctctc gtaacatgca gtcgtaaacg 180

cttgcaatgt cacacgatcy rcattctgat cctyatcrac taatttgtga aagaacaatg 240

ctgctattcc cactttcttc tcacaacaaa gcttcctgac caatgtatta accgtgcgra 300

tccagtactt cttttcaagc ctatctaaga tgaccatagc agtggcatag tcatcttttt 360

ggcaatactt gtgagttaat gtcaacctag ttacttcaca g 401

<210> 79
<211> 23
<212> DNA
<213> Artificial Sequence

<220>
<223> BW 2 Forward Primer

<400> 79
tcaaatccga aagaagagca tac 23

<210> 80
<211> 22
<212> DNA
<213> Artificial Sequence

<220>
<223> BW 2 Reverse Primer

<400> 80
gcagcattgt tctttcacaa at 22

<210> 81
<211> 11
<212> DNA
<213> Artificial Sequence

<220>
<223> BW 2 A allele probe

<220>
<221> misc_feature
<222> (1)..(1)

<220>
<221> misc_feature
<222> (11)..(11)

<400> 81
atgtggatcg t 11

<210> 82
<211> 11
<212> DNA
<213> Artificial Sequence

<220>
<223> BW 2 G allele probe

<220>
<221> misc_feature
<222> (1)..(1)

<220>
<221> misc_feature
<222> (11)..(11)

<400> 82
atgcggatcg t 11

<210> 83
<211> 401
<212> DNA
<213> Fragaria sp.

<400> 83
gaacaatcct gccaactgct gccatagctc aacttttgaa ggctatcgaa aatatacatc 60

cgtcctttcg gtatatttga gctgctagcc aattcctttm gagattctct crgtcaaatc 120

cgaaagaaga gcatacttgt trctctcgta acatgcagtc gtaaacgctt gcaatgtcac 180

acgatcyrca ttctgatcct yatcractaa tttgtgaaag aacaatgctg ctattcccac 240

tttcttctca caacaaagct tcctgaccaa tgtattaacc gtgcgratcc agtacttctt 300

ttcaagccta tctaagatga ccatagcagt ggcatagtca tctttttggc aatacttgtg 360

agttaatgtc aacctagtta cttcacaggg cgacagcccc t 401

<210> 84
<211> 23
<212> DNA
<213> Artificial Sequence

<220>
<223> BW 3 Forward Primer

<400> 84
tcaaatccga aagaagagca tac 23

<210> 85
<211> 22
<212> DNA
<213> Artificial Sequence

<220>
<223> BW 3 Reverse Primer

<400> 85
gcagcattgt tctttcacaa at 22

<210> 86
<211> 14
<212> DNA
<213> Artificial Sequence

<220>
<223> BW 3 T allele probe

<220>
<221> misc_feature
<222> (1)..(1)

<220>
<221> misc_feature
<222> (14)..(14)

<400> 86
tgataaggat caga 14

<210> 87
<211> 12
<212> DNA
<213> Artificial Sequence

<220>

<223>  BW 3 C allele probe

<220>
<221>  misc_feature
<222>  (1)..(1)

<220>
<221>  misc_feature
<222>  (12)..(12)

<400>  87
tgatgaggat ca    12

<210>  88
<211>  401
<212>  DNA
<213>  Fragaria sp.

<400>  88
agcattgtgt ggtcacatgg cttgtcactc tggtgacaag gagaggaagc ttgaagagat    60

tgaggagttt tccgagacta gtgagaagca taagctagtg atggatagtc agtctgatac    120

tgagacctca tcagctcmaa caaagcagag gagggtgtca aagagattga gggasagcwg    180

caactttgaa gttcatggtg rttgttcatc tggttctgga gttgagcaag agcagcagga    240

agtggctatt agtttgatga tgttgtctcg tgattcgggt tataaagggg gtttgaattc    300

gtttggtgag tcttcagata acaattcagt gattttggag ggtaagtcat cttctattga    360

tatgagaatt agtactaaga gaggtctgag ttatggtgct a    401

<210>  89
<211>  20
<212>  DNA
<213>  Artificial Sequence

<220>
<223>  BW 4 Forward Primer

<400>  89
gaggagggtg tcaaagagat    20

<210>  90
<211>  23
<212>  DNA
<213>  Artificial Sequence

<220>
<223>  BW 4 Reverse Primer

<400>  90
cacgagacaa catcatcaaa cta    23

<210>  91
<211>  11
<212>  DNA

<213> Artificial Sequence

<220>
<223> BW 4 G allele probe

<220>
<221> misc_feature
<222> (1)..(1)

<220>
<221> misc_feature
<222> (11)..(11)

<400> 91
caaccaccat g 11

<210> 92
<211> 12
<212> DNA
<213> Artificial Sequence

<220>
<223> BW 4 A allele probe

<220>
<221> misc_feature
<222> (1)..(1)

<220>
<221> misc_feature
<222> (12)..(12)

<400> 92
caatcaccat ga 12

<210> 93
<211> 401
<212> DNA
<213> Fragaria sp.

<400> 93
acgcaacctg ctgcagtgca tatatcacct ccgccaccga aaartactgc tgcatatgaa 60

gcaccggtgt ccaattgcac ctcctctgct gaacacaccc gatcaccatg tcatactcac 120

tcggctctcc caccgcccgc aaatggtggc acagcgagtc aataatcgca ttggccgccg 180

caaactcacc ccgcwgccac raaatgaacc cgtcacgttc atcsgggaac cactgccgcg 240

gcwgctggtg gatctctccg ccagatacag cggctccggc gacactagga tactgcattt 300

tgtccgataa aaccacattc cctgatggca ttgtcattag aaaacccact acacaaaatt 360

ccctaacagc aacaattaat cattccctca ctatatctcc t 401

<210> 94
<211> 20
<212> DNA

<213> Artificial Sequence

<220>
<223> BW 5 Forward Primer

<400> 94
ggcacagcga gtcaataatc 20

<210> 95
<211> 18
<212> DNA
<213> Artificial Sequence

<220>
<223> BW 5 Reverse Primer

<400> 95
cagtggttcc cggatgaa 18

<210> 96
<211> 12
<212> DNA
<213> Artificial Sequence

<220>
<223> BW 5 G allele probe

<220>
<221> misc_feature
<222> (1)..(1)

<220>
<221> misc_feature
<222> (12)..(12)

<400> 96
agccacgaaa tg 12

<210> 97
<211> 13
<212> DNA
<213> Artificial Sequence

<220>
<223> BW 5 A allele probe

<220>
<221> misc_feature
<222> (1)..(1)

<220>
<221> misc_feature
<222> (13)..(13)

<400> 97
agccacaaaa tga 13

<210> 98
<211> 401
<212> DNA
<213> Fragaria sp.

<400> 98
agccaatgga ctcaccaaag aacaaggccg caagtacagc gactgtcaaa ggttgtgaat 60

caattatgac ctgtaatgaa tgccagggtc aaaagggtgt agacatgaaa tgtgatagtt 120

gttaggtaaa gtaactcacg ctgcccagac cagcagacgt yctctgcaaa ccttgagcaa 180

gaaatccttg gaaacaagca kcatcaacga ggccgaagag cgcaatagaa agccaagcgg 240

tgaggccaga aggaaasggg cggcctctgt aggcagcgaa ggcgatgagg aggaagccag 300

caggaatcag acggaaggct gagatgaaga aagggccggt ctttggcaga atgcccttca 360

tggccaccat agccgtaccc caaaagaaga agggagagac c 401

<210> 99
<211> 26
<212> DNA
<213> Artificial Sequence

<220>
<223> BW 6 Forward Primer

<400> 99
tgaaatgtga tagttggtag gtaaag 26

<210> 100
<211> 20
<212> DNA
<213> Artificial Sequence

<220>
<223> BW 6 Reverse Primer

<400> 100
caccgcttgg ctttctattg 20

<210> 101
<211> 12
<212> DNA
<213> Artificial Sequence

<220>
<223> BW 6 G allele probe

<220>
<221> misc_feature
<222> (1)..(1)

<220>
<221> misc_feature
<222> (12)..(12)

<400> 101

```
aagcagcatc aa                                                          12


<210>  102
<211>  14
<212>  DNA
<213>  Artificial Sequence

<220>
<223>  BW 6 T allele probe


<220>
<221>  misc_feature
<222>  (1)..(1)

<220>
<221>  misc_feature
<222>  (14)..(14)

<400>  102
aagcatcatc aacg                                                        14
```

## Description

FIELD OF THE INVENTION

The present invention is directed generally to molecular markers for identification of certain disease resistance and/or agronomic traits in strawberry plants and uses thereof.

BACKGROUND OF THE INVENTION

Strawberry plants (*Fragaria fragaria* × *ananassa*) are grown throughout the world, both commercially and by individuals, for production of strawberry fruit. Breeders of strawberries often seek to develop strawberry plant varieties with improved traits, such as disease resistance traits or agronomic traits.

One disease that is important to the strawberry industry is Fusarium Wilt, which is caused by one or more species of the *Fusarium* fungus, including *Fusarium oxysporum f* sp. *fragariae.* As such, it is desired within the strawberry industry to identify and/or develop varieties of strawberry plants that have natural resistance to these fungal species, and therefore natural resistance to Fusarium Wilt disease. The present application provides molecular markers useful for identifying strawberry plants with improved *Fusarium* resistance and methods of using such markers to identify and/or develop resistant varieties.

Another fungal pathogen that is important to the strawberry industry is *Macrophomina phaseolina*, which causes Macrophomina Crown Rot or Charcoal Rot in infected plants. As such, it is desired within the strawberry industry to identify and/or develop varieties of strawberry plants that have natural resistance to this fungal species, and therefore natural resistance to Macrophomina Crown Rot and Charcoal Rot disease.

Yet another fungal disease that is important to the strawberry industry is Anthracnose Crown Rot, which is caused by several related species of the fugus *Colletotrichum,* including *Collectotrichum acutatum.* As such, it is desired within the strawberry industry to identify and/or develop varieties of strawberry plants that have natural resistance to these fungal species, and therefore natural resistance to the diseases Anthracnose Crown Rot of strawberry.

The present application provides molecular markers useful for identifying strawberry plants with improved *Macrophomina* resistance and/or *Collectotrichum* resistance and methods of using such markers to identify and/or develop resistant varieties.

Two agronomic traits that are important to the strawberry industry are remontancy and berry weight. Remontancy refers to a plant's ability to flower more than once during the course of a growing season, which is often referred to as repeat flowering. In strawberry plants, repeated flowering throughout the growing season results in continual berry production throughout the growing season, which is often a desired trait. With regard to berry weight, increased berry weight is often a desired trait as it results in increased overall yield. The present application provides molecular markers useful for identifying strawberry plants with remontancy and/or increased berry weight and methods of using such markers to identify and/or develop varieties with these agronomic traits.

Further aspects, features and advantages of the present invention will be better appreciated upon a reading of the following detailed description of the invention and claims.

SUMMARY OF INVENTION

Compositions and methods for identifying, selecting, and producing strawberry plants having molecular markers for identification of certain disease resistance, such as resistance to diseases caused by *Fusarium, Macrophomia,* and/or *Colletotrichum,* and/or for identification of agronomic traits, such as remontancy and/or increased berry weight, are provided. Strawberry plants and/or strawberry germplasm and/or parts thereof having molecular markers for identification of *Fusarium* resistance, *Macrophomia* resistance, *Colletotrichum* resistance, remontancy, and/or increased berry weight are also provided.

One or more embodiments relate to at least one, at least two, at least three, at least four, at least five, at least six, or at least seven *Fusarium* resistance markers for a strawberry plant, wherein the *Fusarium* resistance marker(s) are one or more SNPs as shown in Table 1. The SNPs shown in Table 1 represent the alleles conferring improved resistance to disease caused by *Fusarium* infection ("Fusarium resistance allele"). Other embodiments relate to one or two *Macrophomia* resistance markers for a strawberry plant, wherein the *Macrophomia* resistance marker(s) are one or two of the SNPs as shown in Table 2. The SNPs shown in Table 2 represent the alleles conferring improved resistance to disease caused by *Macrophoia* infection ("Macrophomina resistance allele"). Other embodiments relate to the *Colletotrichum* resistance marker for a strawberry plant, wherein the *Colletotrichum* resistance marker is shown in Table 3. The SNP shown in Table 3 represents the allele conferring improved resistance to disease caused by *Colleotrichum* infection ("Colletotrichum resistance allele"). Other embodiments relate to a heterozygous state of the remontancy marker for a strawberry plant, wherein the remontancy marker is shown in Table 4. The SNP shown in Table 4 when in heterozygous state (the strawberry plant has a copy of the SNP with a G at the polymorphism loci and a copy of the SNP with a T at the polymorphism loci) represents the allele conferring improved remontancy ("remontancy allele"). Other embodiments relate to at least one, at least two, at least three, at least four, at least five, or at least six berry weight markers for a strawberry plant, wherein the berry weight marker(s) are one or more SNPs as shown in Table 5. The SNPs shown in Table 5 represent the alleles conferring improved berry weight ("berry weight allele").

One or more embodiments relate to a strawberry plant seed, a strawberry plant, a strawberry plant cultivar, a method for producing a strawberry plant or strawberry plant cultivar, and a method for selecting a strawberry plant or strawberry plant cultivar having at least one, at least two, at least three, at least four, at least five, at least six, or at least seven *Fusarium* resistance markers for a strawberry plant, wherein the *Fusarium* resistance marker(s) are one or more SNPs as shown in Table 1; having one or two *Macrophomia* resistance markers for a strawberry plant, wherein the *Macrophomia* resistance marker(s) are one or two of the SNPs as shown in Table 2; having the *Colletotrichum* resistance marker for a strawberry plant, wherein the *Colletotrichum* resistance marker is shown in Table 3; having the remontancy marker for strawberry plant, wherein the remontancy marker is shown in Table 4; and/or having at least one, at least two, at least three, at least four, at least five, or at least six berry weight markers for a strawberry plant, wherein the berry weight marker(s) are one or more SNPs as shown in Table 5.

One or more embodiments relate to methods for producing or selecting a strawberry plant with improved resistance to *Fusarium* infection, improved resistance to *Macrophomia* infection, improved resistance to *Colletotrichum* infection, improved remontancy, and/or improved berry weight comprising providing an initial population of strawberry plants, obtaining a nucleic acid sample from one or more individuals within said initial population; detecting in each of said nucleic acid samples the presence or absence of one or more SNPs as shown in Tables 1-5, and selecting a strawberry plant from said initial population based on the presence of the one or more SNPs as shown in Table 1-5.

Further embodiments relate to methods for producing or selecting a strawberry plant with improved resistance to *Fusarium* infection, improved resistance to *Macrophomia* infection, improved resistance to *Colletotrichum* infection, improved remontancy, and/or improved berry weight comprising crossing two parental strawberry plants to produce progeny and then detecting in the progeny for the presence or absence of one or more SNPs as shown in Tables 1-5.

Further embodiments relate to methods for producing or selecting a strawberry plant with improved resistance to *Fusarium* infection, improved resistance to *Macrophomia* infection, improved resistance to *Colletotrichum* infection, improved remontancy, and/or improved berry weight comprising crossing a selected strawberry plant that has one or more SNPs as shown in Tables 1-5 with a second strawberry plant to produce progeny plants with improved resistance to *Fusarium* infection, improved resistance to *Macrophomia* infection, improved resistance to *Colletotrichum* infection, improved remontancy, and/or improved berry weight.

Other embodiments relate to methods for producing or selecting a strawberry plant that is homozygous for one or more SNPs as shown in Tables 1-3 and 5.

## BRIEF DESCRIPTION OF THE DRAWINGS

The foregoing summary, as well as the following detailed description of preferred embodiments of the present application, will be better understood when read in conjunction with the appended drawings. It should be understood, however, that the application is not limited to the precise embodiments shown in the drawings.

**FIG. 1A** provides a table of the disease resistance SNPs that were developed.
**FIG. 1B and FIG. 1C** provides tables of the remontancy and berry weight SNPS that were developed.
**FIG. 2A-FIG. 2D** provides the nucleotide sequence of the genome loci containing the identified SNP markers.
**FIG. 3** provides a comparison of selectivity using the *Fusarium* SNP markers identified herein, comparing 2 markers versus 8 markers.
**FIG. 4A-FIG.4D** provides the primer and probe sequences utilized to assay certain identified SNPs.
**FIG. 5A-FIG. 5B** provides the results of a validation study utilizing certain of the identified SNP markers.
**FIG. 6** provides the results of a validation study utilizing the identified SNP markers for berry weight.

## DETAILED DESCRIPTION OF THE INVENTION

### Definitions

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise stated, any numerical values, such as a concentration or a concentration range described herein, are to be understood as being modified in all instances by the term "about." Thus, a numerical value typically includes ± 10% of the recited value. As used herein, the use of a numerical range expressly includes all possible subranges, all individual numerical values within that range, including integers within such ranges and fractions of the values unless the context clearly indicates otherwise.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the invention.

As used herein, the terms "comprises," "comprising," "includes," "including," "has," "having," "contains" or "containing," or any other variation thereof, will be understood to imply the inclusion of a stated item or group of items but not the exclusion of any other item or group of items and are intended to be non-exclusive or open-ended. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

It should also be understood that the terms "about," "approximately," "generally," "substantially" and like terms, used herein when referring to a dimension or characteristic of a component of the preferred invention, indicate that the described dimension/ characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (e.g., rounding, measurement or other systematic errors, manufacturing tolerances, etc.), would not vary the least significant digit.

The term "molecular marker" or "marker" as used herein refers generally to a molecule, including a gene, protein, carbohydrate structure, or glycolipid, the expression of which in or on a mammalian tissue or cell or secreted can be detected by known methods (or methods disclosed herein) and is predictive or can be used to predict (or aid prediction) the presence or absence of a trait, such as improved resistance to certain pathogens or diseases or agronomic characteristics.

A "single nucleotide polymorphism", or "SNP", refers to a single base position in an RNA or DNA molecule (e.g., a polynucleotide), at which different alleles, or alternative nucleotides, exist in a population. The SNP position (interchangeably referred to herein as SNP, SNP site, SNP locus) is usually preceded by and followed by conserved sequences of the allele (e.g., sequences that vary in less than 1/100 or 1/1000 members of the populations). An individual can be homozygous or heterozygous for an allele at each SNP position.

## Molecular markers for identification of certain disease resistance and/or agronomic traits in strawberry plants

### *Fusarium* Resistance Markers for Strawberry Plants

*Fusarium* resistance markers for strawberry plants are identified as one or more of the following SNPs shown in Table 1. The underlined base in the corresponding sequence indicates the polymorphism of the SNP.

Table 1.

| SNP Locus | Sequence |
|---|---|
| PSI-D1 | TGATTGTCTGAGAA (SEQ ID NO: 1) |
| PSI-D2 | TGAAGTATGAATTAA (SEQ ID NO: 2) |
| PSI-D3 | AAGGGCCATGTG (SEQ ID NO: 3) |
| PSI-D4 | CACCGCCTGGTT (SEQ ID NO: 4) |
| PSI-D5 | AACCATCCACAA (SEQ ID NO: 5) |
| PSI-D6 | TGGCCTCCGAG (SEQ ID NO: 6) |
| PSI-D7 | CAGCTTTGGAAT (SEQ ID NO: 7) |

### *Macrophomina* Resistance Markers for Strawberry Plants

*Macrophomina* resistance markers for strawberry plants are identified as one or more of the following SNPs shown in Table 2. The underlined base in the corresponding sequence indicates the polymorphism of the SNP.

**Table 2.**

| SNP Locus | Sequence |
|---|---|
| PSI-D11 | ATGCATAAGATGTG (SEQ ID NO: 8) |
| PSI-D12 | ACTGTAACGACA (SEQ ID NO: 9) |

### *Colletotrichum* Resistance Marker for Strawberry Plants

*Colletotrichum* resistance marker for strawberry plants is identified as the following SNP shown in Table 3. The underlined base in the corresponding sequence indicates the polymorphism of the SNP.

**Table 3.**

| SNP Locus | Sequence |
|---|---|
| PSI-D8 | AGATAGCTAAACTCA (SEQ ID NO: 10) |

### Remontancy Marker for Strawberry Plants

The remontancy marker for strawberry plants is identified as the following SNP shown in Table 4, wherein the remontancy allele is a heterozygous state of the following SNP. The bracketed bases in the corresponding sequence indicate the polymorphism of the SNP.

**Table 4.**

| SNP Locus | Sequence |
|---|---|
| PSI-ROM 1 | GGATC[G/T]GTGAAG (SEQ ID NO: 1 1) |

### Improved Berry Weight Marker for Strawberry Plants

Improved berry weight markers for strawberry plants are identified as one or more of the following SNPs shown in Table 5. The underlined base in the corresponding sequence indicates the polymorphism of the SNP.

**Table 5.**

| SNP Locus | Sequence |
|---|---|
| PSI-BW1 | CTCGCCGTTCTA (SEQ ID NO: 12) |
| PSI-BW2 | GATCYACATTCT (SEQ ID NO: 13) |
| PSI-BW3 | ATCCT<u>T</u>ATCRAC (SEQ ID NO: 14) |
| PSI-BW4 | TGGTGGTTGTTC (SEQ ID NO: 15) |
| PSI-BW5 | GCCAC<u>G</u>AAATGA (SEQ ID NO: 16) |
| PSI-BW6 | AAGCATCATCAA (SEQ ID NO: 17) |

### Exemplified Embodiments:

Embodiment 1. A method for selecting a strawberry plant with improved resistance to *Fusarium* infection, the method comprising:

a. providing an initial population of strawberry plants;
b. obtaining a nucleic acid sample from one or more individuals within said initial population;

c. detecting in each of said nucleic acid samples the presence or absence of two or more alleles conferring improved resistance to disease caused by *Fusarium* infection ("Fusarium resistance allele"), wherein each of the two or more Fusarium resistance alleles is an allele of at least one single nucleotide polymorphism (SNP) marker selected from the SNP loci within SEQ ID NOs: 1-7; and

d. selecting a strawberry plant from said initial population based on the presence of the two or more Fusarium resistance alleles in the nucleic acid sample.

Embodiment 2. A method for selecting a strawberry plant with improved resistance to *Fusarium* infection, the method comprising:

a. crossing two parental strawberry plants to produce a plurality of progeny seed;

b. growing said progeny seed to produce a population of progeny plants;

c. obtaining a nucleic acid sample from one or more individuals within said population of progeny plants;

d. detecting in each of said nucleic acid samples the presence or absence of two or more alleles conferring improved resistance to disease caused by *Fusarium* infection ("Fusarium resistance allele"), wherein each of the two or more Fusarium resistance alleles is an allele of at least one single nucleotide polymorphism (SNP) marker selected from the SNP loci within SEQ ID NOs: 1-7; and

e. selecting a strawberry plant from said population of progeny plants based on the presence of the two or more Fusarium resistance alleles in the nucleic acid sample.

Embodiment 3. A method for obtaining a strawberry plant with improved resistance to *Fusarium* infection, the method comprising:

a. providing an initial population of strawberry plants;

b. obtaining a nucleic acid sample from one or more individuals within said initial population;

c. detecting in each of said nucleic acid samples the presence or absence of two or more alleles conferring improved resistance to disease caused by *Fusarium* infection ("Fusarium resistance allele"), wherein each of the two or more Fusarium resistance alleles is an allele of at least one single nucleotide polymorphism (SNP) loci within SEQ ID NOs:1-7;

d. selecting a strawberry plant from said initial population based on the presence of the two or more Fusarium resistance alleles in the nucleic acid sample; and

e. crossing the selected strawberry plant with a second strawberry plant to produce progeny plants comprising improved resistance to disease caused by *Fusarium* infection.

Embodiment 4. The method of embodiment 3, wherein two strawberry plants are selected from said initial population based on the presence of the two or more Fusarium resistance alleles in the nucleic acid sample for each of the two selected plants, and wherein the two selected plants are crossed with one another to produce progeny plants comprising improved resistance to disease caused by *Fusarium* infection.

Embodiment 5. The method of any one of embodiments 3 or 4, wherein at least one of the two strawberry plants in the cross is homozygous for at least one of the two or more Fusarium resistance alleles.

Embodiment 6. The method of any one of embodiments 3 or 4, wherein both of the two strawberry plants in the cross are homozygous for at least one of the two or more Fusarium resistance alleles.

Embodiment 7. The method of any one of embodiments 1-6, wherein the detection step comprises detecting at least three of the SNP loci within SEQ ID NOs:1-7, optionally at least four of the SNP loci within SEQ ID NOs:1-7, optionally at least five of the SNP loci within SEQ ID NOs:1-7, optionally at least six of the listed SNP loci within SEQ ID NOs:1-7, or optionally at least seven of the SNP loci within SEQ ID NOs:1-7.

Embodiment 8. The method of any one of embodiments 1-7, wherein the detection step further comprises detecting

i) at least one allele conferring improved resistance to disease caused by *Macrophomina* infection ("Macrophomina resistance allele"), wherein each of the at least one Macrophomina resistance alleles is an allele of at least one single nucleotide polymorphism (SNP) loci within SEQ ID NOs:8 and 9;

ii) an allele conferring improved resistance to disease caused by *Colletotrichum* infection ("Colletotrichum resistance allele"), wherein the Colletotrichum resistance allele is an allele of the SNP locus within SEQ ID NO:10;

iii) at least one Macrophomina resitance allele of i) and the Colletotrichum resistance allele of ii); or

iv) both Macrophomina resistance alleles of i) and the Colletotrichum resistance allele of ii).

Embodiment 9. The method of any one of embodiments 1-8, wherein the detection step further comprises detecting the presence or absence of an allele conferring remontancy ("remontancy allele"), wherein the remontancy allele is a heterozygous state at a single nucleotide polymorphism (SNP) locus within SEQ ID NO: 11.

Embodiment 10. A method for selecting a strawberry plant with improved resistance to *Macrophomina* and/or *Colletotrichum* infection, the method comprising:

a. providing an initial population of strawberry plants;
b. obtaining a nucleic acid sample from one or more individuals within said initial population;
c. detecting in each of said nucleic acid samples the presence or absense of two or more alleles conferring improved resistance to disease caused by *Macrophomina* and/or *Colletotrichum* infection ("Macrophomina/Colletotrichum resistance allele"), wherein each of the two or more Macrophomina/Colletotrichum resistance alleles is an allele of at least one single nucleotide polymorphism (SNP) marker selected from the SNP loci within SEQ ID NOs:8-10; and
d. selecting a strawberry plant from said initial population based on the presence of the two or moreMacrophomina/Colletotrichum resistance allelez in the nucleic acid sample.

Embodiment 11. A method for selecting a strawberry plant with improved resistance to *Macrophomina* and/or *Colletotrichum* infection, the method comprising:

a. crossing two parental strawberry plants to produce a plurality of progeny seed;
b. growing said progeny seed to produce a population of progeny plants;
c. obtaining a nucleic acid sample from one or more individuals within said population of progeny plants;
d. detecting in each of said nucleic acid samples the presence or absence of two or more alleles conferring improved resistance to disease caused by *Macrophomina* and/or *Colletotrichum* infection ("Macrophomina/Colletotrichum resistance allele"), wherein each of the two or more Macrophomina/Colletotrichum resistance alleles is an allele of at least one single nucleotide polymorphism (SNP) marker selected from the SNP loci within SEQ ID NOs:8-10; and
e. selecting a strawberry plant from said population of progeny plants based on the presence of the two or more Macrophomina/Colletotrichum resistance alleles in the nucleic acid sample.

Embodiment 12. A method for obtaining a strawberry plant with improved resistance to *Macrophomina* or *Colletotrichum* infection, the method comprising:

a. providing an initial population of strawberry plants;
b. obtaining a nucleic acid sample from one or more individuals within said initial population;
c. detecting in each of said nucleic acid samples the presence or absence of two or more alleles conferring improved resistance to disease caused by *Macrophomina* and/or *Colletotrichum* infection ("Macrophomina/Colletotrichum resistance allele"), wherein each of the two or more Macrophomina/Colletotrichum resistance alleles is an allele of at least one single nucleotide polymorphism (SNP) marker selected from the SNP loci within SEQ ID NOs:8-10;
d. selecting a strawberry plant from said initial population based on the presence of the two or more Macrophomina/Colletotrichum resistance alleles in the nucleic acid sample; and crossing the selected strawberry plant with a second strawberry plant to produce progeny plants comprising improved resistance to disease caused by *Macrophomina* or *Colletotrichum* infection.

Embodiment 13. The method of embodiment 12, wherein two strawberry plants are selected from said initial population based on the presence of the two or more Macrophomina/Colletotrichum resistance alleles in the nucleic acid sample for each of the two selected plants, and wherein the two selected plants are crossed with one another to produce progeny plants comprising improved resistance to disease caused by *Macrophomina* or *Colletotrichum* infection.

Embodiment 14. The method of any one of embodiment 12 or embodiment 13, wherein at least one of the two strawberry plants in the cross is homozygous for at least one of the two or more Macrophomina/Colletotrichum resistance alleles.

Embodiment 15. The method of any one of embodiment 12 or embodiment 13, wherein both of the strawberry plants in the cross are homozygous for at least one of the two or more Macrophomina/Colletotrichum resistance alleles.

Embodiment 16. The method of any one of embodiments 10-15, wherein the detection step comprises detecting three of the SNP loci within SEQ ID NOs:8-10.

Embodiment 17. The method of any one of embodiments 10-16, wherein the detection step further comprises detecting at least one allele conferring improved resistance to disease caused by *Fusarium* infection ("Fusarium resistance allele"), wherein each of the at least one Fusarium resistance alleles is an allele of at least one single nucleotide polymorphism (SNP) loci within SEQ ID NOs:1-7.

Embodiment 18. The method of embodiment 17, wherein the method comprises detecting two or more Fusarium resistance alleles of SEQ ID NOs:1-7, optionally three or more Fusarium resistance alleles of SEQ ID NOs:1-7, optionally four or more Fusarium resistance alleles of SEQ ID NOs:1-7, optionally five or more Fusarium resistance alleles of SEQ ID NOs:1-7, optionally six or more Fusarium resistance alleles of SEQ ID NOs:1-7, optionally seven or more Fusarium resistance alleles of SEQ ID NOs:1-7.

Embodiment 19. The method of any one of embodiment 10-18, wherein the detection step further comprises detecting the presence or absence of an allele conferring remontancy ("remontancy allele"), wherein the remontancy allele is a heterozygous state at a single nucleotide polymorphism (SNP) locus within SEQ ID NO:11.

Embodiment 20. A method for selecting a strawberry plant with improved agronomic traits, the method comprising:

a. providing an initial population of strawberry plants;
b. obtaining a nucleic acid sample from one or more individuals within said initial population;
c. detecting in each of said nucleic acid samples the presence or absence of an allele conferring remontancy ("remontancy allele"), wherein the remontancy allele is a heterozygous state at a single nucleotide polymorphism (SNP) locus within SEQ ID NO:11; and
d. selecting a strawberry plant from said initial population based on the presence of the at remontancy allele in the nucleic acid samples.

Embodiment 21. A method for selecting a strawberry plant with improved agronomic traits, the method comprising:

a. crossing two parental strawberry plants to produce a plurality of progeny seed;
b. growing said progeny seed to produce a population of progeny plants;
c. obtaining a nucleic acid sample from one or more individuals within said population of progeny plants;
d. detecting in each of said nucleic acid samples the presence of an allele conferring remontancy ("remontancy allele"), wherein the remontancy allele is a heterozygous state at a single nucleotide polymorphism (SNP) locus within SEQ ID NO: 11; and
e. selecting a strawberry plant from said population of progeny plants based on the presence of the at remontancy allele in the nucleic acid samples.

Embodiment 22. A method for obtaining a strawberry plant with improved agronomic traits, the method comprising:

a. providing an initial population of strawberry plants;
b. obtaining a nucleic acid sample from one or more individuals within said initial population;
c. detecting in each of said nucleic acid samples the presence of an allele conferring remontancy ("remontancy allele"), wherein the remontancy allele is a heterozygous state at a single nucleotide polymorphism (SNP) locus within SEQ ID NO: 11;
d. selecting a strawberry plant from said population of progeny plants based on the presence of the at remontancy allele in the nucleic acid samples; and
e. crossing the selected strawberry plant with a second strawberry plant to produce progeny plants comprising improved agronomic traits.

Embodiment 23. The method of Embodiment 22, wherein two strawberry plants are selected from said initial population based on the presence of the selected allele in the nucleic acid sample for each of the two selected plants, and wherein the two selected plants are crossed with one another to produce progeny plants comprising improved agronomic traits.

Embodiment 24. The method of any one of Embodiments 20-23, wherein the detection step further comprises detecting in each of said nucleic acid samples the presence or absence of at least one allele conferring increased berry weight ("berry weight allele"), wherein the at least one berry weight allele is an allele of at least one SNP loci within SEQ ID NOs: 12-17.

Embodiment 25. The method of Embodiment 24, wherein the detection step comprises detecting at least two berry weight alleles of SEQ ID NOs: 12-17, optionally at least three berry weight alleles of SEQ ID NOs: 12-17, optionally at least four berry weight alleles of SEQ ID NOs:12-17, optionally at least five berry weight alleles of SEQ ID NOs:12-17, or optionally at least six berry weight alleles of SEQ ID NOs: 12-17.

Embodiment 26. The method of any one of Embodiment 24 or Embodiment 25, wherein at least one of the two strawberry plants in the cross is homozygous for the selected berry weight alleles.

Embodiment 27. The method of any one of Embodiment 24 or Embodiment 25, wherein both of the strawberry plants in the cross are homozygous for the selected berry weight alleles.

## EXAMPLES

### Background of SNP analysis and CIPHER™

By combining genotypic and phenotypic analysis, a SNP marker can be correlated with a particular phenotypic trait, such as a disease resistance trait or agronomic trait. By identifying multiple SNP markers that associate with the same phenotypic trait, it is possible to combine multiple SNP markers into a haplotype that associates even more closely with the desired phenotypic trait.

CIPHER™ is a technology that identifies a set of SNP markers that can be used, either alone or in combination, to identify, with a high likelihood of success, a particular phenotypic trait among individuals in a population. CIPHER™uses sequencing of reduced genomes along with measurements of key traits to generate information rich DNA-based profiles across plant populations using machine learning algorithms. The analysis software then rigorously selects a set of key DNA-based predictors and builds a CIPHER™ algorithm that decrypts the predictors into qualitative haplotype (categorical) values and quantitative (numeric) scores for a specific trait. Diagnostic molecular assays using novel DNA extraction with conventional and isothermal polymerase chain reaction (PCR) are designed by the analysis process and the CIPHER™ is physically validated under a blind test in the laboratory setting.

### Example 1: Developing and implementing Fusarium resistance SNPs and CIPHER™

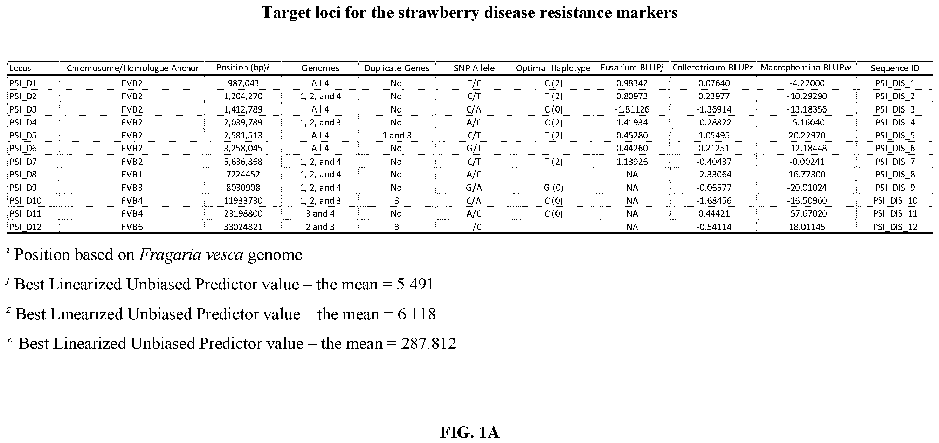

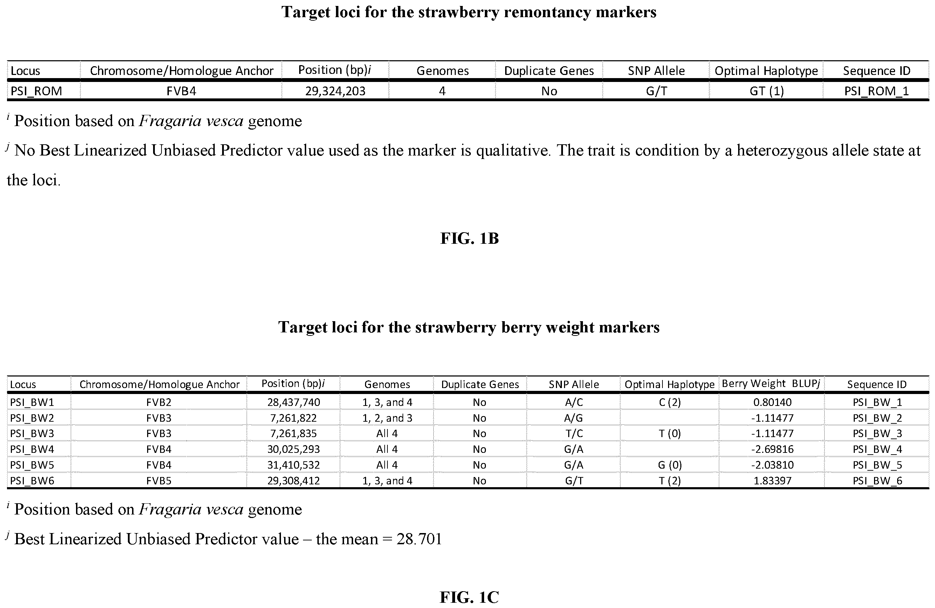

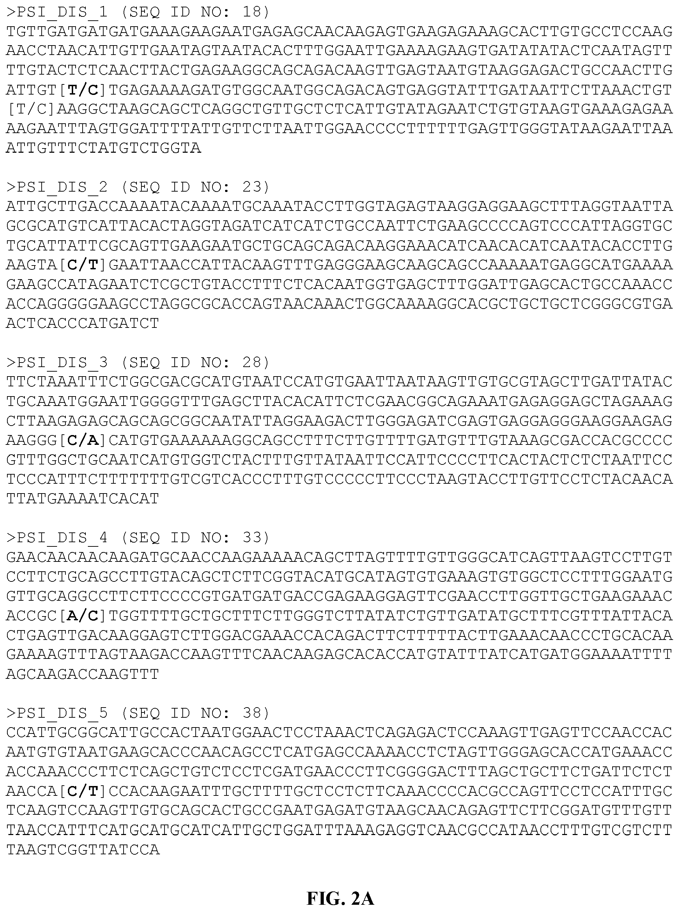

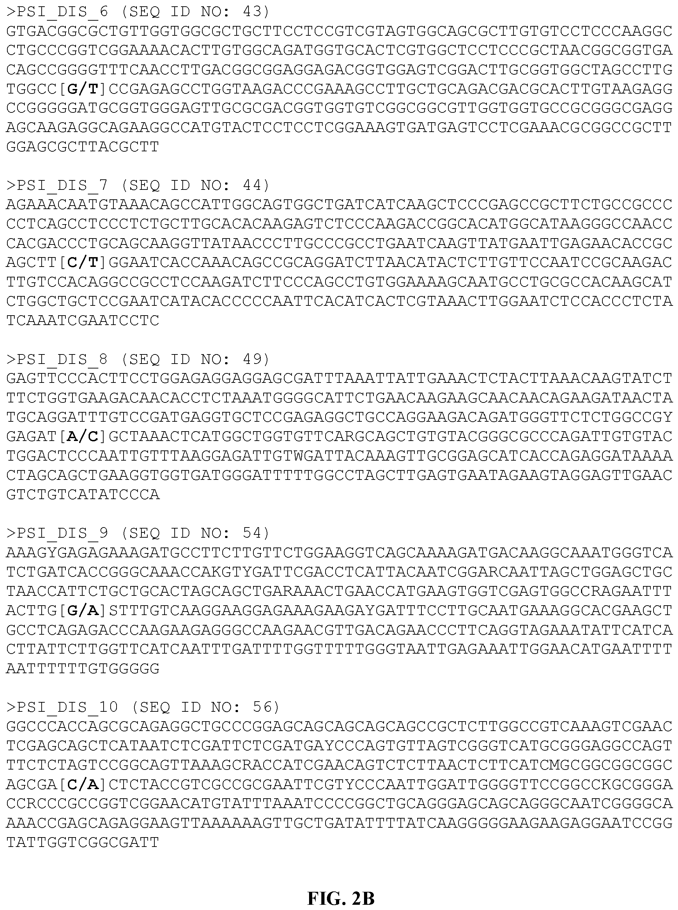

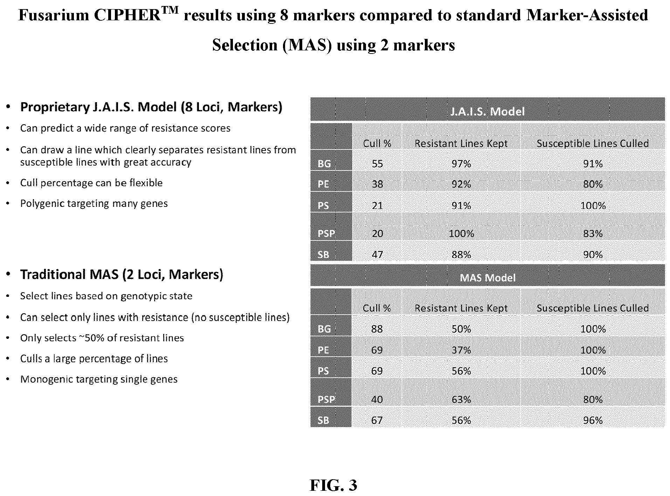

SNPs were identified and CIPHERs™ were developed that are capable of identifying, with high accuracy, strawberry plants that possess improved resistance to *Fusarium* disease, for example disease caused by *Fusarium oxysporum f.* sp. *fragariae*, and, conversely, susceptible plants. The CIPHERs™ are based on decryption algorithms which allow qualitative and quantitative scores to be generated from seven loci or predictors for fusarium (**FIGs 1, 2A, and 2B**). By including an increasing number of SNPs in the selection criteria, and depending on which combined set of SNPs is selected, it is possible to have a much more selective determination of resistant versus susceptible lines than can be achieved by using only one or a few SNPs. Fusarium resistance haplotypes and scores were predicted for over 1,800 strawberry lines from five different breeding programs. Comparing the predicted scores to 300 known measured fusarium resistance scores in a blind test, a threshold could be discerned in each of the 5 programs which separated resistant lines from susceptible lines with over 88% accuracy on average. A traditional marker-assisted-selection method based on two markers that can accurately identify resistant lines based on genotypic state (present/absent) was used to select resistant lines. This method was only able to select just over 52% of the total known resistant lines on average (**FIG. 3**).

The disease CIPHERs™ were developed by carefully selecting a training set of lines. The training set was evaluated for resistance to *Fusarium* sp., *M. phaseolina, and C. acutatum* under controlled greenhouse conditions over three years (2017, 2018, 2020). The mean disease severity (phenotypes) for each line in the training set were combined with the corresponding cipheralleles (genotypes) and the software developed a preliminary prediction algorithm using its proprietary adaptive learning network. The prediction algorithm was then validated using both cross validation with the appropriate hold-out strategy for the trait type and physical validation. The algorithm was then optimized using a proprietary selection network to identify the smallest subset of predictors or alleles that accurately predict the trait.

The Fusarium CIPHER™ Algorithm:

$$\overline{y} = \mu + i^{1*\alpha} + (i^{2*\rho})^2 + i^{3*\gamma} + i^{4*\delta} + i^{5*\varepsilon} + i^{6\theta} + i^{7*\beta}$$

where predicted trait or attribute value ( $\overline{y}$ ) is a derivative of the dynamic mean ($\mu$) plus the numeric state of the CIPHER™ allele (*ith*) times the corresponding J.A.I.S. linearized unbiased prediction value for the *ith* allele ($\alpha$, $\beta$, $\gamma$, $\delta$, $\varepsilon$, $\theta$, $\rho$) was used to impute values for over 1,800 lines in the programs based on the following calculus:

$$\overline{y} = 5.491 + i^{1*[0.983]} + \left(i^{2*[0.810]}\right)^2 + i^{3*[-1.813]} + i^{4*[1.419]} + i^{5*[0.453]} + i^{6[0.443]} + i^{7*[1.139]}$$

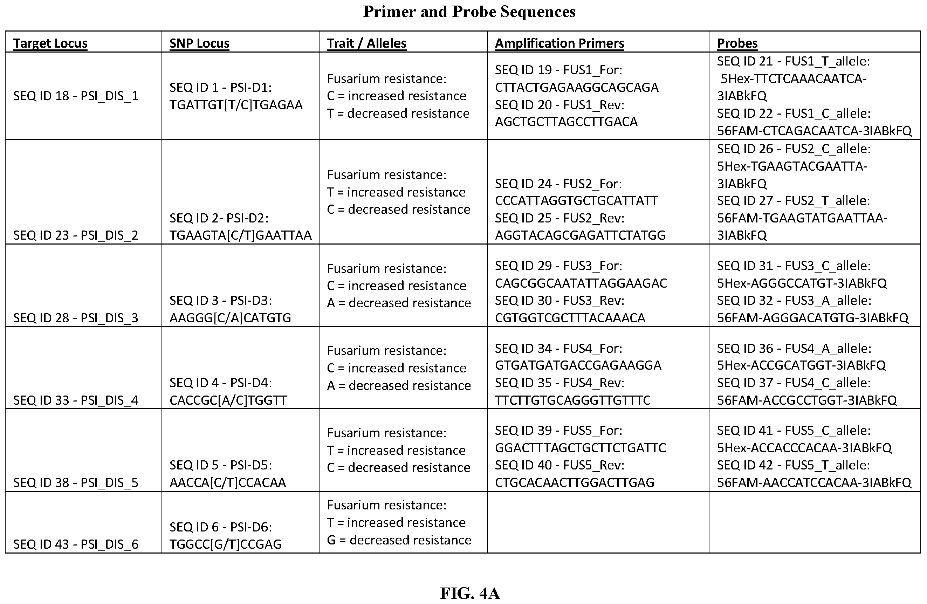

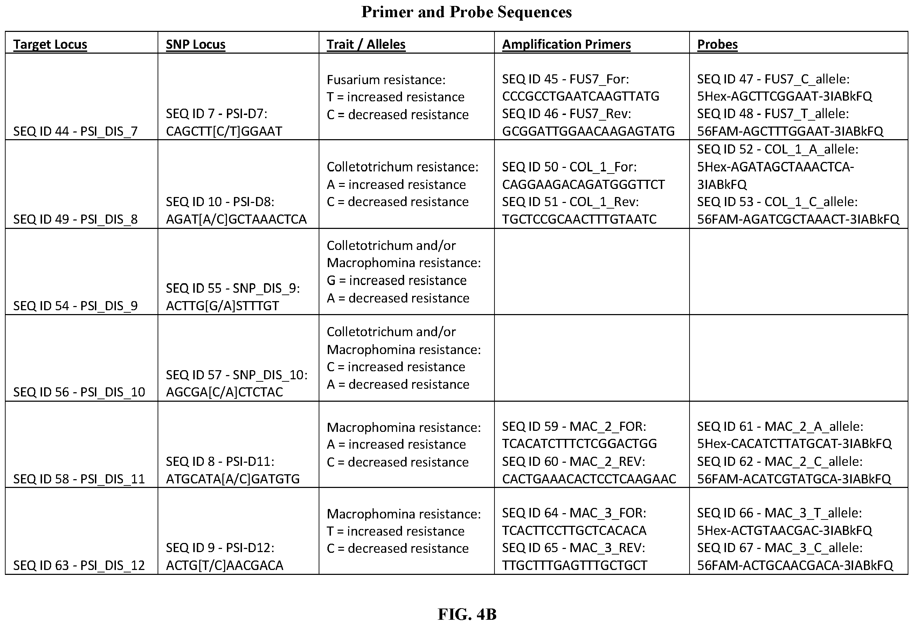

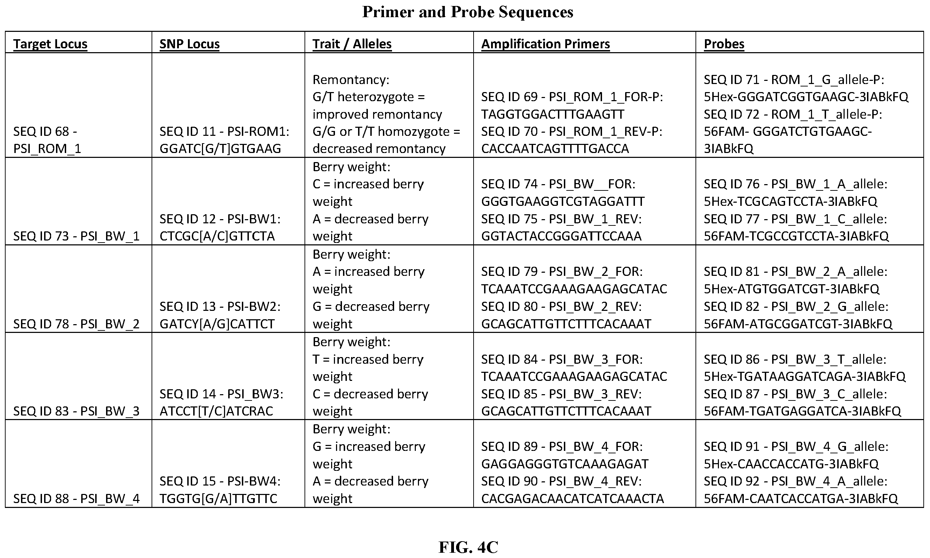

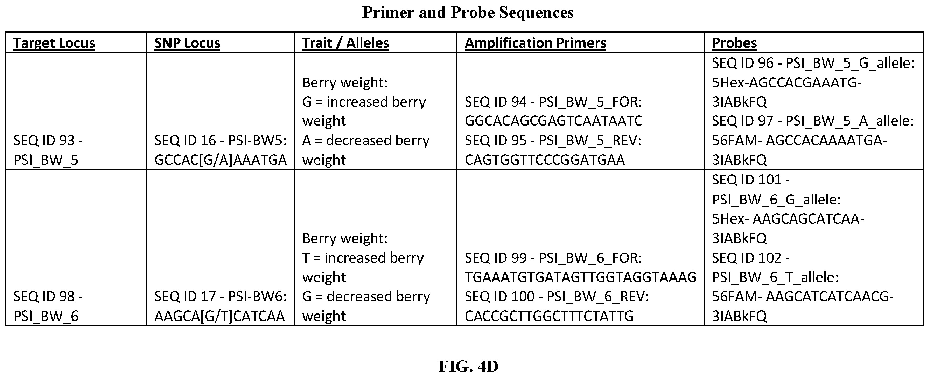

The sequence information from each locus (**FIG. 2A and FIG.2B**) was used to create the Trait CIPHER™ Assays using molecular beacon technology (**FIGs. 4A-4D**). In brief, forward and reverse primers were designed to amplify the target region containing the single nucleotide polymorphism (SNP). Two different forward primers were designed, one specific to each of the SNP alleles in question. The forward primers are differentiated by a short sequence on the 5' end that does not match the template DNA but is homologous to a secondary probe sequence that contains a specific fluorophore molecule. Once the sequence is amplified during Polymerase Chain Reaction (PCR), the probe corresponding to the SNP allele in the sample is incorporated into the growing template and the fluorophore is released. The free fluorophore is then read using a spectrophotometer and the SNP allele is differentiated.

The imputed trait or attribute values of a test set (N = 300 lines) representing five breeding programs that were not screened in the greenhouse experiments were compared to actual disease severity measurements using the Trait CIPHER™ Assays. The comparisons yielded an accuracy of 88% or greater, showing greater than 97% accuracy for fusarium. When comparing this result to the traditional marker-assisted-selection method currently being deployed, the Fusarium CIPHER™ was on average 40.8% more accurate.

The fusarium CIPHER™ was used to cull out fusarium susceptible lines across the breeding programs (N~150,000 seedlings) prior to transplanting from the greenhouse to the field. In brief, $F_1$ seeds were planted in trays and incubated in the greenhouse. Three-millimeter leaf disks were cut from each seedling and placed in a 96-well plate maintaining the line identification using a plate record key. The plates were sent in for Trait CIPHER™ sequencing and the data was uploaded to a database. A workflow utilizing the Fusarium CIPHER™ Algorithm deciphered the data for each line into predicted fusarium haplotype and severity scores. A culling tool was utilized to set the minimum fusarium severity score desired and view the overall metrics for percent cull, maximum/minimum severity, and unique severity scores for each cross. Once the desired culling percent was determined via evaluation of the metrices, the wells in each tray containing a line to be culled was identified using a proprietary algorithm embedded in the workflow. In addition, individual tray maps were constructed to guide physically removing the unwanted lines from the trays while consolidating the good lines.

The overall accuracy of the CIPHER™ algorithm was re-evaluated based on the assumed segregation ratios for each cross and by testing selected lines in the greenhouse under artificial *Fusarium* disease pressure. Overall, the expected segregation ratios were calculated for the crosses and the selected lines fit the expected disease response greater than 98% of the time.

A close evaluation of the haplotype representation of all seven allele states that make up the Fusarium CIPHER™ revealed that the optimal allele state:

$$\bar{y}\ 5.491 + 2*0.983 + (2*0.810)^2 + 0*\text{-}1.812 + 2*1.419 + 2*0.453 + 2*0.443 + 2*1.139$$

does not exist in the breeding programs. The theoretical resistance of the optimal haplotype would be 1.5 times greater than currently possible. The program fixes the alleles utilizing the Trait CIPHER™. The Fusarium CIPHER™ can be used to screen a global strawberry population to confirm the uniqueness of this optimal haplotype.

**Example 2: Developing and implementing Macrophomina/Colletotrichum resistance SNPs and CIPHER™**

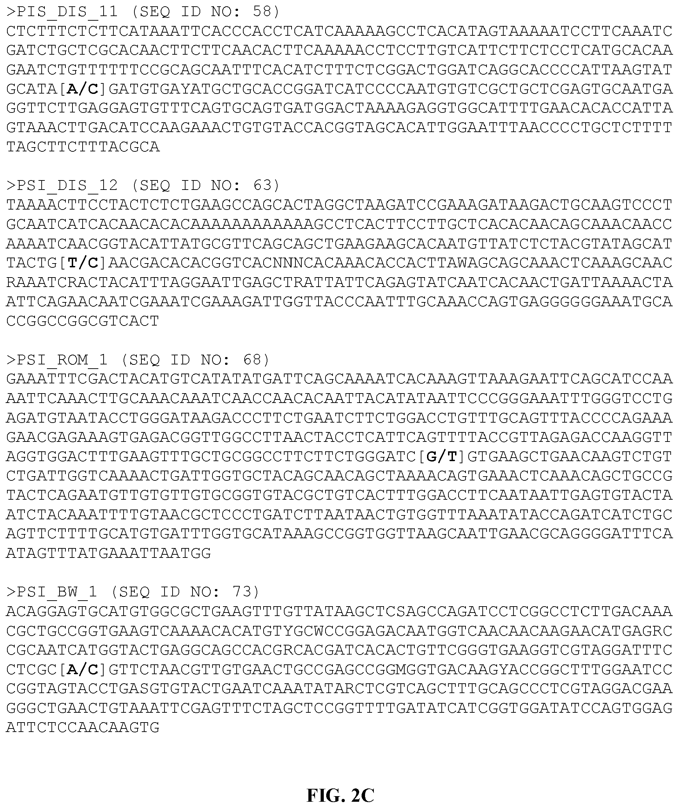

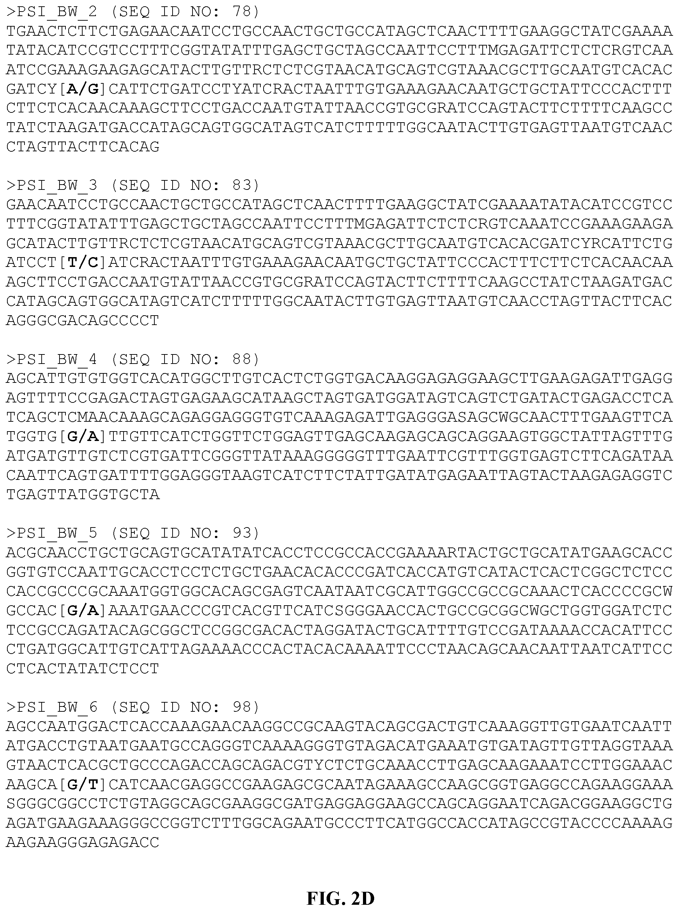

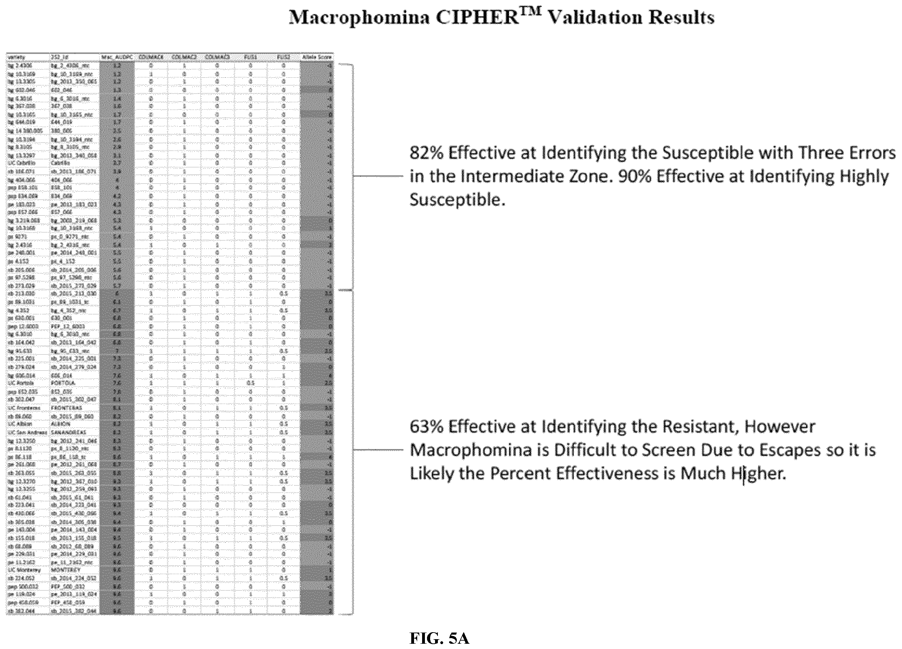

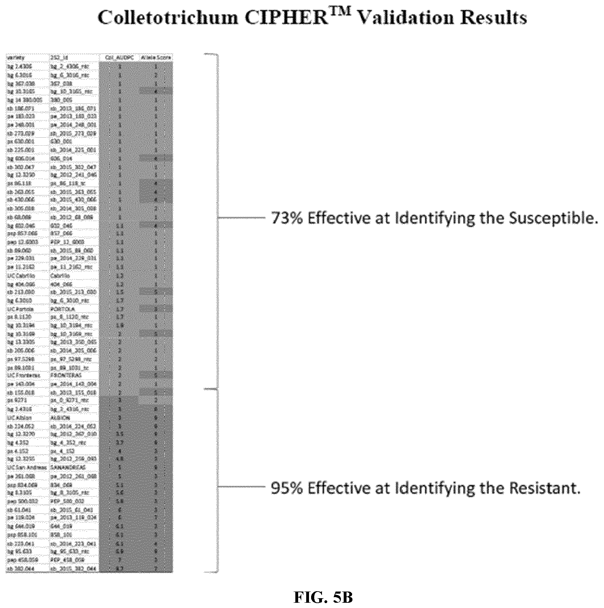

SNPs were identified and CIPHERs™ were developed that are capable of identifying, with high accuracy, strawberry plants that possess improved resistance to *Macrophomina and/or Colletotrichum* disease, for example disease caused by *Macrophomina phaseolina* or *Colletotrichum acutatum,* and, conversely, susceptible plants. The CIPHERs™ are based on decryption algorithms which allow qualitative and quantitative scores to be generated from five loci or predictors for macrophomina and colletotrichum (**FIGs. 1, 2B, and 2C**). By including an increasing number of SNPs in the selection criteria, and depending on which combined set of SNPs is selected, it is possible to have a much more selective determination of resistant versus susceptible lines than can be achieved by using only one or a few SNPs. Macrophomina and colletotrichum resistance haplotypes and scores were predicted for over 1,800 strawberry lines from five different breeding programs. Haplotypes and allele scores generated from the prediction algorithm were compared across 70 lines for both macrophomina and colletotrichum in a 10 blind test. The results indicated the macrophomina CIPHER™ could identify susceptible lines with and accuracy of 82% and resistant lines with and accuracy of 63% (**FIG. 5A**), while the colletotrichum CIPHER™ could identify susceptible lines with and accuracy 73% of and resistant lines with and accuracy of 95% (**FIG. 5B**). The disease CIPHERs™ were developed by carefully selecting a training set of lines. The training set was evaluated for resistance to *Fusarium* sp., *M. phaseolina, and C. acutatum* under controlled greenhouse conditions over three years (2017, 2018, 2020). The mean disease severity (phenotypes) for each line in the training set were combined with the corresponding cipheralleles (genotypes) and the software developed a preliminary prediction algorithm using its proprietary adaptive learning network. The prediction algorithm was then validated using both cross validation with the appropriate hold-out strategy for the trait type and physical validation. The algorithm was then optimized using a proprietary selection network to identify the smallest subset of predictors or alleles (**FIG. 2**) that accurately predict the trait.

Macrophomina:

$$y = 287.812 + j^{1\,*\,-4\cdot22} + i^{2\,*\,-10\cdot29} + i^{3\,*\,-13\cdot18} + i^{4\,*\,-5\cdot16} + i^{5\,*\,20\cdot23} + i^{6\,*\,-12\cdot18} + i^{7\,*\,-0\cdot002} + i^{8\,*\,16\cdot77} + i^{9\,*\,-20\cdot01} + i^{10\,*\,-16\cdot51} + i^{11\,*\,-57\cdot67} + i^{12\,*\,18\cdot01}$$

Colletotrichum:

$$y = 6.118 + j^{1\,*\,0\cdot08} + i^{2\,*\,0\cdot24} + i^{3\,*\,-1\cdot37} + i^{4\,*\,-0\cdot29} + i^{5\,*\,1\cdot05} + i^{6\,*\,0\cdot21} + i^{7\,*\,-0\cdot40} + i^{8\,*\,-2\cdot33} + i^{9\,*\,-0\cdot66} + i^{10\,*\,-1\cdot68} + i^{11\,*\,0\cdot44} + i^{12\,*\,-0\cdot54}$$

The sequence information from each locus (**FIG 2**) was used to create the Trait CIPHER™ Assays using molecular beacon technology (**FIGs. 4A-4D**). In brief, forward and reverse primes were designed to amplify the target region containing the single nucleotide polymorphism (SNP). Two different forward primers were designed, one specific to each of the SNP alleles in question. The forward primers are differentiated by a short sequence on the 5' end that does not match the template DNA but is homologous to a secondary probe sequence that contains a specific fluorophore molecule. Once the sequence is amplified during Polymerase Chain Reaction (PCR), the probe corresponding to the SNP allele in the sample is incorporated into the growing template and the fluorophore is released. The free fluorophore is then read using a spectrophotometer and the SNP allele is differentiated.

The imputed trait or attribute values of a test set (N = 300 lines) representing five breeding programs that were not screened in the greenhouse experiments were compared to actual disease severity measurements using the Trait CIPHER™ Assays.

The optimal allele states were calculated for:

Macrophomina

$$y{=}287.812 + j^{1\,(0*-4.22)} + i^{2\,(0*-10.29)} + i^{3\,(0*-13.18)} + i^{4\,(0*-5.16)} + i^{5\,(2*20.23)} + i6^{\,(0*-12.18)} + i7^{\,(0*-0.002)} + i8^{\,(2*16.77)} + i9^{\,(0*-20.01)} + i10^{\,(0*-116.51)} + i11^{\,(0*57.67)} + i^{12\,(2*18.01)}$$

Colletotrichum

$$y{=}6.118 + j^{1\,(2*0.08)} + i^{2\,(2*0.24)} + i^{3\,(0*-1.37)} + i^{4\,(0*-0.29)} + i^{5\,(2*1.05)} + i6^{\,(2*0.21)} + i7^{\,(0*-0.40)} + i8^{\,(0*-2.33)} + i9^{\,(0*-0.66)} + i10^{\,(0*-1.68)} + i11^{\,(2*0.44)} + i^{12\,(0*-0.54)}$$

And were not found within the programs.

## Example 3: Developing and implementing agronomic trait SNPs and CIPHER™

SNPs were identified and CIPHERs™ were developed that are capable of identifying, with high accuracy, strawberry plants that express remontancy and increased berry weight, and, conversely, those plants that do not. By including an increasing number of SNPs in the selection criteria, and depending on which combined set of SNPs is selected, it is possible to have a much more selective determination of lines possessing the desired agronomic traits than can be achieved by using only one or a few SNPs. A single locus was discovered where in the heterozygous state conditioned remontancy (**FIGs 1, 2C and 2D**). The CIPHER™ was tested across over 500 lines under blind test and was shown to have an accuracy of over 90%.

The Berry weight CIPHER™ included six loci that associate with this phenotypic trait (**FIGs 1 and 2**):

$$\bar{y} = \mu + j^{1\,*\,\alpha} + i^{2\,*\,\rho} + i^{3\,*\,\gamma} + i^{4\,*\,\delta} + i^{5\,*\,\varepsilon} + i^{6\,*\,\theta}$$

where predicted trait or attribute value ( $\bar{y}$ ) is a derivative of the dynamic J.A.I.S. mean ($\mu$) plus the numeric state of the CIPHER™ allele (*ith*) times the corresponding J.A.I.S. linearized unbiased prediction value for the *ith* allele ($\alpha$, $\beta$, $\gamma$, $\delta$, $\varepsilon$, $\theta$) was used to impute values for over 1,800 lines in the PSI programs based on the following calculus:

$$\bar{y} = 28.71 + j^{1\,2\,*\,0\cdot80} + i^{2\,*\,-1\cdot11} + i^{3\,*\,-1\cdot11} + i^{4\,*\,-2\cdot70} + i^{5\,*\,-2\cdot04} + i^{6\,*\,1\cdot83}$$

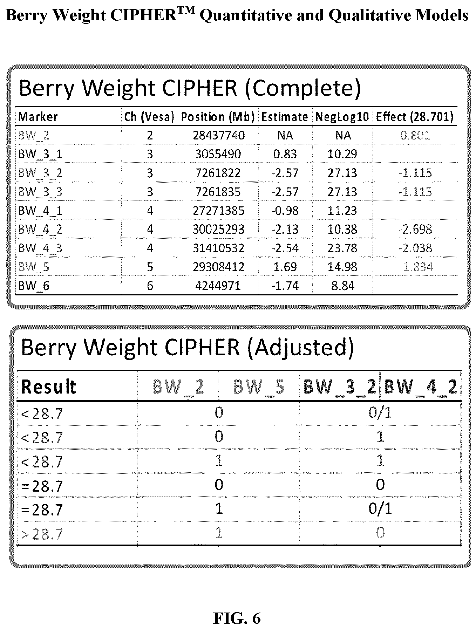

In addition, the optimal allele state was used which allowed three classes of discrimination including greater than, equal to, or less than 28-gram berries with an accuracy of 85% (FIG. 6). The optimal allele state:

$$y=28.71+i^{1\ (0*0.80)}+i^{2\ (0*-1.11)}+i^{3\ (0*-1.11)}+i^{4\ (0*-2.70)}+i^{5\ (0*-2.04)}+i^{6\ (2*1.83)}$$

was not found across the breeding programs.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the present description.

All documents cited herein are incorporated by reference.

**Claims**

**1.** A method for selecting a strawberry plant with improved resistance to *Fusarium* infection, the method comprising:

a. providing an initial population of strawberry plants;
b. obtaining a nucleic acid sample from one or more individuals within said initial population;
c. detecting in each of said nucleic acid samples the presence or absence of two or more alleles conferring improved resistance to disease caused by *Fusarium* infection ("Fusarium resistance allele"), wherein each of the two or more Fusarium resistance alleles is an allele of at least one single nucleotide polymorphism (SNP) marker selected from the SNP loci within SEQ ID NOs: 1-7; and
d. selecting a strawberry plant from said initial population based on the presence of the two or more Fusarium resistance alleles in the nucleic acid sample.

**2.** A method for selecting a strawberry plant with improved resistance to *Fusarium* infection, the method comprising:

a. crossing two parental strawberry plants to produce a plurality of progeny seed;
b. growing said progeny seed to produce a population of progeny plants;
c. obtaining a nucleic acid sample from one or more individuals within said population of progeny plants;
d. detecting in each of said nucleic acid samples the presence or absence of two or more alleles conferring improved resistance to disease caused by *Fusarium* infection ("Fusarium resistance allele"), wherein each of the two or more Fusarium resistance alleles is an allele of at least one single nucleotide polymorphism (SNP) marker selected from the SNP loci within SEQ ID NOs: 1-7; and
e. selecting a strawberry plant from said population of progeny plants based on the presence of the two or more Fusarium resistance alleles in the nucleic acid sample.

**3.** A method for obtaining a strawberry plant with improved resistance to *Fusarium* infection, the method comprising:

a. providing an initial population of strawberry plants;
b. obtaining a nucleic acid sample from one or more individuals within said initial population;
c. detecting in each of said nucleic acid samples the presence or absence of two or more alleles conferring improved resistance to disease caused by *Fusarium* infection ("Fusarium resistance allele"), wherein each of the two or more Fusarium resistance alleles is an allele of at least one single nucleotide polymorphism (SNP) loci within SEQ ID NOs:1-7;
d. selecting a strawberry plant from said initial population based on the presence of the two or more Fusarium resistance alleles in the nucleic acid sample; and

e. crossing the selected strawberry plant with a second strawberry plant to produce progeny plants comprising improved resistance to disease caused by *Fusarium* infection.

**4.** The method of Claim 3, wherein two strawberry plants are selected from said initial population based on the presence of the two or more Fusarium resistance alleles in the nucleic acid sample for each of the two selected plants, and wherein the two selected plants are crossed with one another to produce progeny plants comprising improved resistance to disease caused by *Fusarium* infection.

**5.** The method of any one of Claim 3 or Claim 4, wherein at least one of the two strawberry plants in the cross is homozygous for at least one of the two or more Fusarium resistance alleles.

**6.** The method of any one of Claims 3 or Claim 4, wherein both of the two strawberry plants in the cross are homozygous for at least one of the two or more Fusarium resistance alleles.

**7.** The method of any one of Claims 1-6, wherein the detection step comprises detecting at least three of the SNP loci within SEQ ID NOs:1-7, optionally at least four of the SNP loci within SEQ ID NOs:1-7, optionally at least five of the SNP loci within SEQ ID NOs:1-7, optionally at least six of the listed SNP loci within SEQ ID NOs:1-7, or optionally at least seven of the SNP loci within SEQ ID NOs:1-7.

**8.** The method of any one of Claims 1-7, wherein the detection step further comprises detecting

i) at least one allele conferring improved resistance to disease caused by *Macrophomina* infection ("Macrophomina resistance allele"), wherein each of the at least one Macrophomina resistance alleles is an allele of at least one single nucleotide polymorphism (SNP) loci within SEQ ID NOs:8 and 9;
ii) an allele conferring improved resistance to disease caused by *Colletotrichum* infection ("Colletotrichum resistance allele"), wherein the Colletotrichum resistance allele is an allele of the SNP locus within SEQ ID NO:10;
iii) at least one Macrophomina resitance allele of i) and the Colletotrichum resistance allele of ii); or
iv) both Macrophomina resistance alleles of i) and the Colletotrichum resistance allele of ii).

**9.** The method of any one of Claims 1-8, wherein the detection step further comprises detecting the presence or absence of an allele conferring remontancy ("remontancy allele"), wherein the remontancy allele is a heterozygous state at a single nucleotide polymorphism (SNP) locus within SEQ ID NO: 11.

## Target loci for the strawberry disease resistance markers

| Locus | Chromosome/Homologue Anchor | Position (bp)*i* | Genomes | Duplicate Genes | SNP Allele | Optimal Haplotype | Fusarium BLUP*j* | Colletotricum BLUPz | Macrophomina BLUP*w* | Sequence ID |
|---|---|---|---|---|---|---|---|---|---|---|
| PSI_D1 | FVB2 | 987,043 | All 4 | No | T/C | C (2) | 0.98342 | 0.07640 | -4.22000 | PSI_DIS_1 |
| PSI_D2 | FVB2 | 1,204,270 | 1, 2, and 4 | No | C/T | T (2) | 0.80973 | 0.23977 | -10.29290 | PSI_DIS_2 |
| PSI_D3 | FVB2 | 1,412,789 | All 4 | No | C/A | C (0) | -1.81126 | -1.36914 | -13.18356 | PSI_DIS_3 |
| PSI_D4 | FVB2 | 2,039,789 | 1, 2, and 3 | No | A/C | C (2) | 1.41934 | -0.28822 | -5.16040 | PSI_DIS_4 |
| PSI_D5 | FVB2 | 2,581,513 | All 4 | 1 and 3 | C/T | T (2) | 0.45280 | 1.05495 | 20.22970 | PSI_DIS_5 |
| PSI_D6 | FVB2 | 3,258,045 | All 4 | No | G/T | | 0.44260 | 0.21251 | -12.18448 | PSI_DIS_6 |
| PSI_D7 | FVB2 | 5,636,868 | 1, 2, and 4 | No | C/T | T (2) | 1.13926 | -0.40437 | -0.00241 | PSI_DIS_7 |
| PSI_D8 | FVB1 | 7224452 | 1, 2, and 4 | No | A/C | | NA | -2.33064 | 16.77300 | PSI_DIS_8 |
| PSI_D9 | FVB3 | 8030908 | 1, 2, and 4 | No | G/A | G (0) | NA | -0.06577 | -20.01024 | PSI_DIS_9 |
| PSI_D10 | FVB4 | 11933730 | 1, 2, and 3 | 3 | C/A | C (0) | NA | -1.68456 | -16.50960 | PSI_DIS_10 |
| PSI_D11 | FVB4 | 23198800 | 3 and 4 | No | A/C | C (0) | NA | 0.44421 | -57.67020 | PSI_DIS_11 |
| PSI_D12 | FVB6 | 33024821 | 2 and 3 | 3 | T/C | | NA | -0.54114 | 18.01145 | PSI_DIS_12 |

$^{i}$ Position based on *Fragaria vesca* genome

$^{j}$ Best Linearized Unbiased Predictor value – the mean = 5.491

$^{z}$ Best Linearized Unbiased Predictor value – the mean = 6.118

$^{w}$ Best Linearized Unbiased Predictor value – the mean = 287.812

**FIG. 1A**

**Target loci for the strawberry remontancy markers**

| Locus | Chromosome/Homologue Anchor | Position (bp)[i] | Genomes | Duplicate Genes | SNP Allele | Optimal Haplotype | Sequence ID |
|---|---|---|---|---|---|---|---|
| PSI_ROM | FVB4 | 29,324,203 | 4 | No | G/T | GT (1) | PSI_ROM_1 |

[i] Position based on *Fragaria vesca* genome

[j] No Best Linearized Unbiased Predictor value used as the marker is qualitative. The trait is condition by a heterozygous allele state at the loci.

**FIG. 1B**

**Target loci for the strawberry berry weight markers**

| Locus | Chromosome/Homologue Anchor | Position (bp)[i] | Genomes | Duplicate Genes | SNP Allele | Optimal Haplotype | Berry Weight BLUP[j] | Sequence ID |
|---|---|---|---|---|---|---|---|---|
| PSI_BW1 | FVB2 | 28,437,740 | 1, 3, and 4 | No | A/C | C (2) | 0.80140 | PSI_BW_1 |
| PSI_BW2 | FVB3 | 7,261,822 | 1, 2, and 3 | No | A/G | | -1.11477 | PSI_BW_2 |
| PSI_BW3 | FVB3 | 7,261,835 | All 4 | No | T/C | T (0) | -1.11477 | PSI_BW_3 |
| PSI_BW4 | FVB4 | 30,025,293 | All 4 | No | G/A | | -2.69816 | PSI_BW_4 |
| PSI_BW5 | FVB4 | 31,410,532 | All 4 | No | G/A | G (0) | -2.03810 | PSI_BW_5 |
| PSI_BW6 | FVB5 | 29,308,412 | 1, 3, and 4 | No | G/T | T (2) | 1.83397 | PSI_BW_6 |

[i] Position based on *Fragaria vesca* genome

[j] Best Linearized Unbiased Predictor value – the mean = 28.701

**FIG. 1C**

>PSI_DIS_1 (SEQ ID NO: 18)
TGTTGATGATGATGAAAGAAGAATGAGAGCAACAAGAGTGAAGAGAAAGCACTTGTGCCTCCAAG
AACCTAACATTGTTGAATAGTAATACACTTTGGAATTGAAAAGAAGTGATATATACTCAATAGTT
TTGTACTCTCAACTTACTGAGAAGGCAGCAGACAAGTTGAGTAATGTAAGGAGACTGCCAACTTG
ATTGT[**T/C**]TGAGAAAAGATGTGGCAATGGCAGACAGTGAGGTATTTGATAATTCTTAAACTGT
[T/C]AAGGCTAAGCAGCTCAGGCTGTTGCTCTCATTGTATAGAATCTGTGTAAGTGAAAGAGAA
AAGAATTTAGTGGATTTTATTGTTCTTAATTGGAACCCCTTTTTTGAGTTGGGTATAAGAATTAA
ATTGTTTCTATGTCTGGTA

>PSI_DIS_2 (SEQ ID NO: 23)
ATTGCTTGACCAAAATACAAAATGCAAATACCTTGGTAGAGTAAGGAGGAAGCTTTAGGTAATTA
GCGCATGTCATTACACTAGGTAGATCATCATCTGCCAATTCTGAAGCCCCAGTCCCATTAGGTGC
TGCATTATTCGCAGTTGAAGAATGCTGCAGCAGACAAGGAAACATCAACACATCAATACACCTTG
AAGTA[**C/T**]GAATTAACCATTACAAGTTTGAGGGAAGCAAGCAGCCAAAAATGAGGCATGAAAA
GAAGCCATAGAATCTCGCTGTACCTTTCTCACAATGGTGAGCTTTGGATTGAGCACTGCCAAACC
ACCAGGGGGAAGCCTAGGCGCACCAGTAACAAACTGGCAAAAGGCACGCTGCTGCTCGGGCGTGA
ACTCACCCATGATCT

>PSI_DIS_3 (SEQ ID NO: 28)
TTCTAAATTTCTGGCGACGCATGTAATCCATGTGAATTAATAAGTTGTGCGTAGCTTGATTATAC
TGCAAATGGAATTGGGGTTTGAGCTTACACATTCTCGAACGGCAGAAATGAGAGGAGCTAGAAAG
CTTAAGAGAGCAGCAGCGGCAATATTAGGAAGACTTGGGAGATCGAGTGAGGAGGGAAGGAAGAG
AAGGG[**C/A**]CATGTGAAAAAAGGCAGCCTTTCTTGTTTTGATGTTTGTAAAGCGACCACGCCCC
GTTTGGCTGCAATCATGTGGTCTACTTTGTTATAATTCCATTCCCCTTCACTACTCTCTAATTCC
TCCCATTTCTTTTTTTGTCGTCACCCTTTGTCCCCCTTCCCTAAGTACCTTGTTCCTCTACAACA
TTATGAAAATCACAT

>PSI_DIS_4 (SEQ ID NO: 33)
GAACAACAACAAGATGCAACCAAGAAAAACAGCTTAGTTTTGTTGGGCATCAGTTAAGTCCTTGT
CCTTCTGCAGCCTTGTACAGCTCTTCGGTACATGCATAGTGTGAAAGTGTGGCTCCTTTGGAATG
GTTGCAGGCCTTCTTCCCCGTGATGATGACCGAGAAGGAGTTCGAACCTTGGTTGCTGAAGAAAC
ACCGC[**A/C**]TGGTTTTGCTGCTTTCTTGGGTCTTATATCTGTTGATATGCTTTCGTTTATTACA
CTGAGTTGACAAGGAGTCTTGGACGAAACCACAGACTTCTTTTTACTTGAAACAACCCTGCACAA
GAAAAGTTTAGTAAGACCAAGTTTCAACAAGAGCACACCATGTATTTATCATGATGGAAAATTTT
AGCAAGACCAAGTTT

>PSI_DIS_5 (SEQ ID NO: 38)
CCATTGCGGCATTGCCACTAATGGAACTCCTAAACTCAGAGACTCCAAAGTTGAGTTCCAACCAC
AATGTGTAATGAAGCACCCAACAGCCTCATGAGCCAAAACCTCTAGTTGGGAGCACCATGAAACC
ACCAAACCCTTCTCAGCTGTCTCCTCGATGAACCCTTCGGGGACTTTAGCTGCTTCTGATTCTCT
AACCA[**C/T**]CCACAAGAATTTGCTTTTGCTCCTCTTCAAACCCCACGCCAGTTCCTCCATTTGC
TCAAGTCCAAGTTGTGCAGCACTGCCGAATGAGATGTAAGCAACAGAGTTCTTCGGATGTTTGTT
TAACCATTTCATGCATGCATCATTGCTGGATTTAAAGAGGTCAACGCCATAACCTTTGTCGTCTT
TAAGTCGGTTATCCA

**FIG. 2A**

>PSI_DIS_6 (SEQ ID NO: 43)
GTGACGGCGCTGTTGGTGGCGCTGCTTCCTCCGTCGTAGTGGCAGCGCTTGTGTCCTCCCAAGGC
CTGCCCGGTCGGAAAACACTTGTGGCAGATGGTGCACTCGTGGCTCCTCCCGCTAACGGCGGTGA
CAGCCGGGGTTTCAACCTTGACGGCGGAGGAGACGGTGGAGTCGGACTTGCGGTGGCTAGCCTTG
TGGCC[**G/T**]CCGAGAGCCTGGTAAGACCCGAAAGCCTTGCTGCAGACGACGCACTTGTAAGAGG
CCGGGGGATGCGGTGGGAGTTGCGCGACGGTGGTGTCGGCGGCGTTGGTGGTGCCGCGGGCGAGG
AGCAAGAGGCAGAAGGCCATGTACTCCTCCTCGGAAAGTGATGAGTCCTCGAAACGCGGCCGCTT
GGAGCGCTTACGCTT

>PSI_DIS_7 (SEQ ID NO: 44)
AGAAACAATGTAAACAGCCATTGGCAGTGGCTGATCATCAAGCTCCCGAGCCGCTTCTGCCGCCC
CCTCAGCCTCCCTCTGCTTGCACACAAGAGTCTCCCAAGACCGGCACATGGCATAAGGGCCAACC
CACGACCCTGCAGCAAGGTTATAACCCTTGCCCGCCTGAATCAAGTTATGAATTGAGAACACCGC
AGCTT[**C/T**]GGAATCACCAAACAGCCGCAGGATCTTAACATACTCTTGTTCCAATCCGCAAGAC
TTGTCCACAGGCCGCCTCCAAGATCTTCCCAGCCTGTGGAAAAGCAATGCCTGCGCCACAAGCAT
CTGGCTGCTCCGAATCATACACCCCCAATTCACATCACTCGTAAACTTGGAATCTCCACCCTCTA
TCAAATCGAATCCTC

>PSI_DIS_8 (SEQ ID NO: 49)
GAGTTCCCACTTCCTGGAGAGGAGGAGCGATTTAAATTATTGAAACTCTACTTAAACAAGTATCT
TTCTGGTGAAGACAACACCTCTAAATGGGGCATTCTGAACAAGAAGCAACAACAGAAGATAACTA
TGCAGGATTTGTCCGATGAGGTGCTCCGAGAGGCTGCCAGGAAGACAGATGGGTTCTCTGGCCGY
GAGAT[**A/C**]GCTAAACTCATGGCTGGTGTTCARGCAGCTGTGTACGGGCGCCCAGATTGTGTAC
TGGACTCCCAATTGTTTAAGGAGATTGTWGATTACAAAGTTGCGGAGCATCACCAGAGGATAAAA
CTAGCAGCTGAAGGTGGTGATGGGATTTTTGGCCTAGCTTGAGTGAATAGAAGTAGGAGTTGAAC
GTCTGTCATATCCCA

>PSI_DIS_9 (SEQ ID NO: 54)
AAAGYGAGAGAAAGATGCCTTCTTGTTCTGGAAGGTCAGCAAAAGATGACAAGGCAAATGGGTCA
TCTGATCACCGGGCAAACCAKGTYGATTCGACCTCATTACAATCGGARCAATTAGCTGGAGCTGC
TAACCATTCTGCTGCACTAGCAGCTGARAAACTGAACCATGAAGTGGTCGAGTGGCCRAGAATTT
ACTTG[**G/A**]STTTGTCAAGGAAGGAGAAAGAAGAYGATTTCCTTGCAATGAAAGGCACGAAGCT
GCCTCAGAGACCCAAGAAGAGGGCCAAGAACGTTGACAGAACCCTTCAGGTAGAAATATTCATCA
CTTATTCTTGGTTCATCAATTTGATTTTGGTTTTTGGGTAATTGAGAAATTGGAACATGAATTTT
AATTTTTTGTGGGGG

>PSI_DIS_10 (SEQ ID NO: 56)
GGCCCACCAGCGCAGAGGCTGCCCGGAGCAGCAGCAGCAGCCGCTCTTGGCCGTCAAAGTCGAAC
TCGAGCAGCTCATAATCTCGATTCTCGATGAYCCCAGTGTTAGTCGGGTCATGCGGGAGGCCAGT
TTCTCTAGTCCGGCAGTTAAAGCRACCATCGAACAGTCTCTTAACTCTTCATCMGCGGCGGCGGC
AGCGA[**C/A**]CTCTACCGTCGCCGCGAATTCGTYCCCAATTGGATTGGGGTTCCGGCCKGCGGGA
CCRCCCGCCGGTCGGAACATGTATTTAAATCCCCGGCTGCAGGGAGCAGCAGGGCAATCGGGGCA
AAACCGAGCAGAGGAAGTTAAAAAAGTTGCTGATATTTTATCAAGGGGGAAGAAGAGGAATCCGG
TATTGGTCGGCGATT

# FIG. 2B

>PIS_DIS_11 (SEQ ID NO: 58)
CTCTTTCTCTTCATAAATTCACCCACCTCATCAAAAAGCCTCACATAGTAAAAATCCTTCAAATC
GATCTGCTCGCACAACTTCTTCAACACTTCAAAAACCTCCTTGTCATTCTTCTCCTCATGCACAA
GAATCTGTTTTTTCCGCAGCAATTTCACATCTTTCTCGGACTGGATCAGGCACCCCATTAAGTAT
GCATA[**A/C**]GATGTGAYATGCTGCACCGGATCATCCCCAATGTGTCGCTGCTCGAGTGCAATGA
GGTTCTTGAGGAGTGTTTCAGTGCAGTGATGGACTAAAAGAGGTGGCATTTTGAACACACCATTA
GTAAACTTGACATCCAAGAAACTGTGTACCACGGTAGCACATTGGAATTTAACCCCTGCTCTTTT
TAGCTTCTTTACGCA

>PSI_DIS_12 (SEQ ID NO: 63)
TAAAACTTCCTACTCTCTGAAGCCAGCACTAGGCTAAGATCCGAAAGATAAGACTGCAAGTCCCT
GCAATCATCACAACACACAAAAAAAAAAAAAGCCTCACTTCCTTGCTCACACAACAGCAAACAACC
AAAATCAACGGTACATTATGCGTTCAGCAGCTGAAGAAGCACAATGTTATCTCTACGTATAGCAT
TACTG[**T/C**]AACGACACACGGTCACNNNCACAAACACCACTTAWAGCAGCAAACTCAAAGCAAC
RAAATCRACTACATTTAGGAATTGAGCTRATTATTCAGAGTATCAATCACAACTGATTAAAACTA
ATTCAGAACAATCGAAATCGAAAGATTGGTTACCCAATTTGCAAACCAGTGAGGGGGGAAATGCA
CCGGCCGGCGTCACT

>PSI_ROM_1 (SEQ ID NO: 68)
GAAATTTCGACTACATGTCATATATGATTCAGCAAAATCACAAAGTTAAAGAATTCAGCATCCAA
AATTCAAACTTGCAAACAAATCAACCAACACAATTACATATAATTCCCGGGAAATTTGGGTCCTG
AGATGTAATACCTGGGATAAGACCCTTCTGAATCTTCTGGACCTGTTTGCAGTTTACCCCAGAAA
GAACGAGAAAGTGAGACGGTTGGCCTTAACTACCTCATTCAGTTTTACCGTTAGAGACCAAGGTT
AGGTGGACTTTGAAGTTTGCTGCGGCCTTCTTCTGGGATC[**G/T**]GTGAAGCTGAACAAGTCTGT
CTGATTGGTCAAAACTGATTGGTGCTACAGCAACAGCTAAAACAGTGAAACTCAAACAGCTGCCG
TACTCAGAATGTTGTGTTGTGCGGTGTACGCTGTCACTTTGGACCTTCAATAATTGAGTGTACTA
ATCTACAAATTTTGTAACGCTCCCTGATCTTAATAACTGTGGTTTAAATATACCAGATCATCTGC
AGTTCTTTTGCATGTGATTTGGTGCATAAAGCCGGTGGTTAAGCAATTGAACGCAGGGGATTTCA
ATAGTTTATGAAATTAATGG

>PSI_BW_1 (SEQ ID NO: 73)
ACAGGAGTGCATGTGGCGCTGAAGTTTGTTATAAGCTCSAGCCAGATCCTCGGCCTCTTGACAAA
CGCTGCCGGTGAAGTCAAAACACATGTYGCWCCGGAGACAATGGTCAACAACAAGAACATGAGRC
CGCAATCATGGTACTGAGGCAGCCACGRCACGATCACACTGTTCGGGTGAAGGTCGTAGGATTTC
CTCGC[**A/C**]GTTCTAACGTTGTGAACTGCCGAGCCGGMGGTGACAAGYACCGGCTTTGGAATCC
CGGTAGTACCTGASGTGTACTGAATCAAATATARCTCGTCAGCTTTGCAGCCCTCGTAGGACGAA
GGGCTGAACTGTAAATTCGAGTTTCTAGCTCCGGTTTTGATATCATCGGTGGATATCCAGTGGAG
ATTCTCCAACAAGTG

**FIG. 2C**

>PSI_BW_2 (SEQ ID NO: 78)
TGAACTCTTCTGAGAACAATCCTGCCAACTGCTGCCATAGCTCAACTTTTGAAGGCTATCGAAAA
TATACATCCGTCCTTTCGGTATATTTGAGCTGCTAGCCAATTCCTTTMGAGATTCTCTCRGTCAA
ATCCGAAAGAAGAGCATACTTGTTRCTCTCGTAACATGCAGTCGTAAACGCTTGCAATGTCACAC
GATCY[**A/G**]CATTCTGATCCTYATCRACTAATTTGTGAAAGAACAATGCTGCTATTCCCACTTT
CTTCTCACAACAAAGCTTCCTGACCAATGTATTAACCGTGCGRATCCAGTACTTCTTTTCAAGCC
TATCTAAGATGACCATAGCAGTGGCATAGTCATCTTTTTGGCAATACTTGTGAGTTAATGTCAAC
CTAGTTACTTCACAG

>PSI_BW_3 (SEQ ID NO: 83)
GAACAATCCTGCCAACTGCTGCCATAGCTCAACTTTTGAAGGCTATCGAAAATATACATCCGTCC
TTTCGGTATATTTGAGCTGCTAGCCAATTCCTTTMGAGATTCTCTCRGTCAAATCCGAAAGAAGA
GCATACTTGTTRCTCTCGTAACATGCAGTCGTAAACGCTTGCAATGTCACACGATCYRCATTCTG
ATCCT[**T/C**]ATCRACTAATTTGTGAAAGAACAATGCTGCTATTCCCACTTTCTTCTCACAACAA
AGCTTCCTGACCAATGTATTAACCGTGCGRATCCAGTACTTCTTTTCAAGCCTATCTAAGATGAC
CATAGCAGTGGCATAGTCATCTTTTTGGCAATACTTGTGAGTTAATGTCAACCTAGTTACTTCAC
AGGGCGACAGCCCCT

>PSI_BW_4 (SEQ ID NO: 88)
AGCATTGTGTGGTCACATGGCTTGTCACTCTGGTGACAAGGAGAGGAAGCTTGAAGAGATTGAGG
AGTTTTCCGAGACTAGTGAGAAGCATAAGCTAGTGATGGATAGTCAGTCTGATACTGAGACCTCA
TCAGCTCMAACAAAGCAGAGGAGGGTGTCAAAGAGATTGAGGGASAGCWGCAACTTTGAAGTTCA
TGGTG[**G/A**]TTGTTCATCTGGTTCTGGAGTTGAGCAAGAGCAGCAGGAAGTGGCTATTAGTTTG
ATGATGTTGTCTCGTGATTCGGGTTATAAAGGGGGTTTGAATTCGTTTGGTGAGTCTTCAGATAA
CAATTCAGTGATTTTGGAGGGTAAGTCATCTTCTATTGATATGAGAATTAGTACTAAGAGAGGTC
TGAGTTATGGTGCTA

>PSI_BW_5 (SEQ ID NO: 93)
ACGCAACCTGCTGCAGTGCATATATCACCTCCGCCACCGAAAARTACTGCTGCATATGAAGCACC
GGTGTCCAATTGCACCTCCTCTGCTGAACACACCCGATCACCATGTCATACTCACTCGGCTCTCC
CACCGCCCGCAAATGGTGGCACAGCGAGTCAATAATCGCATTGGCCGCCGCAAACTCACCCCGCW
GCCAC[**G/A**]AAATGAACCCGTCACGTTCATCSGGGAACCACTGCCGCGGCWGCTGGTGGATCTC
TCCGCCAGATACAGCGGCTCCGGCGACACTAGGATACTGCATTTTGTCCGATAAAACCACATTCC
CTGATGGCATTGTCATTAGAAAACCCACTACACAAAATTCCCTAACAGCAACAATTAATCATTCC
CTCACTATATCTCCT

>PSI_BW_6 (SEQ ID NO: 98)
AGCCAATGGACTCACCAAAGAACAAGGCCGCAAGTACAGCGACTGTCAAAGGTTGTGAATCAATT
ATGACCTGTAATGAATGCCAGGGTCAAAAGGGTGTAGACATGAAATGTGATAGTTGTTAGGTAAA
GTAACTCACGCTGCCCAGACCAGCAGACGTYCTCTGCAAACCTTGAGCAAGAAATCCTTGGAAAC
AAGCA[**G/T**]CATCAACGAGGCCGAAGAGCGCAATAGAAAGCCAAGCGGTGAGGCCAGAAGGAAA
SGGGCGGCCTCTGTAGGCAGCGAAGGCGATGAGGAGGAAGCCAGCAGGAATCAGACGGAAGGCTG
AGATGAAGAAAGGGCCGGTCTTTGGCAGAATGCCCTTCATGGCCACCATAGCCGTACCCCAAAAG
AAGAAGGGAGAGACC

**FIG. 2D**

**Fusarium CIPHER™ results using 8 markers compared to standard Marker-Assisted Selection (MAS) using 2 markers**

- **Proprietary J.A.I.S. Model (8 Loci, Markers)**
- Can predict a wide range of resistance scores
- Can draw a line which clearly separates resistant lines from susceptible lines with great accuracy
- Cull percentage can be flexible
- Polygenic targeting many genes

- **Traditional MAS (2 Loci, Markers)**
- Select lines based on genotypic state
- Can select only lines with resistance (no susceptible lines)
- Only selects ~50% of resistant lines
- Culls a large percentage of lines
- Monogenic targeting single genes

| | J.A.I.S. Model | | |
|---|---|---|---|
| | Cull % | Resistant Lines Kept | Susceptible Lines Culled |
| BG | 55 | 97% | 91% |
| PE | 38 | 92% | 80% |
| PS | 21 | 91% | 100% |
| PSP | 20 | 100% | 83% |
| SB | 47 | 88% | 90% |

| | MAS Model | | |
|---|---|---|---|
| | Cull % | Resistant Lines Kept | Susceptible Lines Culled |
| BG | 88 | 50% | 100% |
| PE | 69 | 37% | 100% |
| PS | 69 | 56% | 100% |
| PSP | 40 | 63% | 80% |
| SB | 67 | 56% | 96% |

**FIG. 3**

## Primer and Probe Sequences

| Target Locus | SNP Locus | Trait / Alleles | Amplification Primers | Probes |
|---|---|---|---|---|
| SEQ ID 18 - PSI_DIS_1 | SEQ ID 1 - PSI-D1:<br>TGATTGT[T/C]TGAGAA | Fusarium resistance:<br>C = increased resistance<br>T = decreased resistance | SEQ ID 19 - FUS1_For:<br>CTTACTGAGAAGGCAGCAGA<br>SEQ ID 20 - FUS1_Rev:<br>AGCTGCTTAGCCTTGACA | SEQ ID 21 - FUS1_T_allele:<br>5Hex-TTCTCAAACAATCA-3IABkFQ<br>SEQ ID 22 - FUS1_C_allele:<br>56FAM-CTCAGACAATCA-3IABkFQ |
| SEQ ID 23 - PSI_DIS_2 | SEQ ID 2- PSI-D2:<br>TGAAGTA[C/T]GAATTAA | Fusarium resistance:<br>T = increased resistance<br>C = decreased resistance | SEQ ID 24 - FUS2_For:<br>CCCATTAGGTGCTGCATTATT<br>SEQ ID 25 - FUS2_Rev:<br>AGGTACAGCGAGATTCTATGG | SEQ ID 26 - FUS2_C_allele:<br>5Hex-TGAAGTACGAATTA-3IABkFQ<br>SEQ ID 27 - FUS2_T_allele:<br>56FAM-TGAAGTATGAATTAA-3IABkFQ |
| SEQ ID 28 - PSI_DIS_3 | SEQ ID 3 - PSI-D3:<br>AAGGG[C/A]CATGTG | Fusarium resistance:<br>C = increased resistance<br>A = decreased resistance | SEQ ID 29 - FUS3_For:<br>CAGCGGCAATATTAGGAAGAC<br>SEQ ID 30 - FUS3_Rev:<br>CGTGGTCGCTTTACAAACA | SEQ ID 31 - FUS3_C_allele:<br>5Hex-AGGGCCATGT-3IABkFQ<br>SEQ ID 32 - FUS3_A_allele:<br>56FAM-AGGGACATGTG-3IABkFQ |
| SEQ ID 33 - PSI_DIS_4 | SEQ ID 4 - PSI-D4:<br>CACCGC[A/C]TGGTT | Fusarium resistance:<br>C = increased resistance<br>A = decreased resistance | SEQ ID 34 - FUS4_For:<br>GTGATGATGACCGAGAAGGA<br>SEQ ID 35 - FUS4_Rev:<br>TTCTTGTGCAGGGTTGTTTC | SEQ ID 36 - FUS4_A_allele:<br>5Hex-ACCGCATGGT-3IABkFQ<br>SEQ ID 37 - FUS4_C_allele:<br>56FAM-ACCGCCTGGT-3IABkFQ |
| SEQ ID 38 - PSI_DIS_5 | SEQ ID 5 - PSI-D5:<br>AACCA[C/T]CCACAA | Fusarium resistance:<br>T = increased resistance<br>C = decreased resistance | SEQ ID 39 - FUS5_For:<br>GGACTTTAGCTGCTTCTGATTC<br>SEQ ID 40 - FUS5_Rev:<br>CTGCACAACTTGGACTTGAG | SEQ ID 41 - FUS5_C_allele:<br>5Hex-ACCACCCACAA-3IABkFQ<br>SEQ ID 42 - FUS5_T_allele:<br>56FAM-AACCATCCACAA-3IABkFQ |
| SEQ ID 43 - PSI_DIS_6 | SEQ ID 6 - PSI-D6:<br>TGGCC[G/T]CCGAG | Fusarium resistance:<br>T = increased resistance<br>G = decreased resistance | | |

**FIG. 4A**

## Primer and Probe Sequences

| Target Locus | SNP Locus | Trait / Alleles | Amplification Primers | Probes |
|---|---|---|---|---|
| SEQ ID 44 - PSI_DIS_7 | SEQ ID 7 - PSI-D7: CAGCTT[C/T]GGAAT | Fusarium resistance:<br>T = increased resistance<br>C = decreased resistance | SEQ ID 45 - FUS7_For: CCCGCCTGAATCAAGTTATG<br>SEQ ID 46 - FUS7_Rev: GCGGATTGGAACAAGAGTATG | SEQ ID 47 - FUS7_C_allele: 5Hex-AGCTTCGGAAT-3IABkFQ<br>SEQ ID 48 - FUS7_T_allele: 56FAM-AGCTTTGGAAT-3IABkFQ |
| SEQ ID 49 - PSI_DIS_8 | SEQ ID 10 - PSI-D8: AGAT[A/C]GCTAAACTCA | Colletotrichum resistance:<br>A = increased resistance<br>C = decreased resistance | SEQ ID 50 - COL_1_For: CAGGAAGACAGATGGGTTCT<br>SEQ ID 51 - COL_1_Rev: TGCTCCGCAACTTTGTAATC | SEQ ID 52 - COL_1_A_allele: 5Hex-AGATAGCTAAACTCA-3IABkFQ<br>SEQ ID 53 - COL_1_C_allele: 56FAM-AGATCGCTAAACT-3IABkFQ |
| SEQ ID 54 - PSI_DIS_9 | SEQ ID 55 - SNP_DIS_9: ACTTG[G/A]STTTGT | Colletotrichum and/or Macrophomina resistance:<br>G = increased resistance<br>A = decreased resistance | | |
| SEQ ID 56 - PSI_DIS_10 | SEQ ID 57 - SNP_DIS_10: AGCGA[C/A]CTCTAC | Colletotrichum and/or Macrophomina resistance:<br>C = increased resistance<br>A = decreased resistance | | |
| SEQ ID 58 - PSI_DIS_11 | SEQ ID 8 - PSI-D11: ATGCATA[A/C]GATGTG | Macrophomina resistance:<br>A = increased resistance<br>C = decreased resistance | SEQ ID 59 - MAC_2_FOR: TCACATCTTTCTCGGACTGG<br>SEQ ID 60 - MAC_2_REV: CACTGAAACACTCCTCAAGAAC | SEQ ID 61 - MAC_2_A_allele: 5Hex-CACATCTTATGCAT-3IABkFQ<br>SEQ ID 62 - MAC_2_C_allele: 56FAM-ACATCGTATGCA-3IABkFQ |
| SEQ ID 63 - PSI_DIS_12 | SEQ ID 9 - PSI-D12: ACTG[T/C]AACGACA | Macrophomina resistance:<br>T = increased resistance<br>C = decreased resistance | SEQ ID 64 - MAC_3_FOR: TCACTTCCTTGCTCACACA<br>SEQ ID 65 - MAC_3_REV: TTGCTTTGAGTTTGCTGCT | SEQ ID 66 - MAC_3_T_allele: 5Hex-ACTGTAACGAC-3IABkFQ<br>SEQ ID 67 - MAC_3_C_allele: 56FAM-ACTGCAACGACA-3IABkFQ |

**FIG. 4B**

**Primer and Probe Sequences**

| Target Locus | SNP Locus | Trait / Alleles | Amplification Primers | Probes |
|---|---|---|---|---|
| SEQ ID 68 - PSI_ROM_1 | SEQ ID 11 - PSI-ROM1: GGATC[G/T]GTGAAG | Remontancy: G/T heterozygote = improved remontancy G/G or T/T homozygote = decreased remontancy | SEQ ID 69 - PSI_ROM_1_FOR-P: TAGGTGGACTTTGAAGTT SEQ ID 70 - PSI_ROM_1_REV-P: CACCAATCAGTTTTGACCA | SEQ ID 71 - ROM_1_G_allele-P: 5Hex-GGGATCGGTGAAGC-3IABkFQ SEQ ID 72 - ROM_1_T_allele-P: 56FAM- GGGATCTGTGAAGC-3IABkFQ |
| SEQ ID 73 - PSI_BW_1 | SEQ ID 12 - PSI-BW1: CTCGC[A/C]GTTCTA | Berry weight: C = increased berry weight A = decreased berry weight | SEQ ID 74 - PSI_BW__FOR: GGGTGAAGGTCGTAGGATTT SEQ ID 75 - PSI_BW_1_REV: GGTACTACCGGGATTCCAAA | SEQ ID 76 - PSI_BW_1_A_allele: 5Hex-TCGCAGTCCTA-3IABkFQ SEQ ID 77 - PSI_BW_1_C_allele: 56FAM-TCGCCGTCCTA-3IABkFQ |
| SEQ ID 78 - PSI_BW_2 | SEQ ID 13 - PSI-BW2: GATCY[A/G]CATTCT | Berry weight: A = increased berry weight G = decreased berry weight | SEQ ID 79 - PSI_BW_2_FOR: TCAAATCCGAAAGAAGAGCATAC SEQ ID 80 - PSI_BW_2_REV: GCAGCATTGTTCTTTCACAAAT | SEQ ID 81 - PSI_BW_2_A_allele: 5Hex-ATGTGGATCGT-3IABkFQ SEQ ID 82 - PSI_BW_2_G_allele: 56FAM-ATGCGGATCGT-3IABkFQ |
| SEQ ID 83 - PSI_BW_3 | SEQ ID 14 - PSI_BW3: ATCCT[T/C]ATCRAC | Berry weight: T = increased berry weight C = decreased berry weight | SEQ ID 84 - PSI_BW_3_FOR: TCAAATCCGAAAGAAGAGCATAC SEQ ID 85 - PSI_BW_3_REV: GCAGCATTGTTCTTTCACAAAT | SEQ ID 86 - PSI_BW_3_T_allele: 5Hex-TGATAAGGATCAGA-3IABkFQ SEQ ID 87 - PSI_BW_3_C_allele: 56FAM-TGATGAGGATCA-3IABkFQ |
| SEQ ID 88 - PSI_BW_4 | SEQ ID 15 - PSI-BW4: TGGTG[G/A]TTGTTC | Berry weight: G = increased berry weight A = decreased berry weight | SEQ ID 89 - PSI_BW_4_FOR: GAGGAGGGTGTCAAAGAGAT SEQ ID 90 - PSI_BW_4_REV: CACGAGACAACATCATCAAACTA | SEQ ID 91 - PSI_BW_4_G_allele: 5Hex-CAACCACCATG-3IABkFQ SEQ ID 92 - PSI_BW_4_A_allele: 56FAM-CAATCACCATGA-3IABkFQ |

FIG. 4C

**Primer and Probe Sequences**

| Target Locus | SNP Locus | Trait / Alleles | Amplification Primers | Probes |
|---|---|---|---|---|
| SEQ ID 93 - PSI_BW_5 | SEQ ID 16 - PSI-BW5: GCCAC[G/A]AAATGA | Berry weight:<br>G = increased berry weight<br>A = decreased berry weight | SEQ ID 94 - PSI_BW_5_FOR: GGCACAGCGAGTCAATAATC<br>SEQ ID 95 - PSI_BW_5_REV: CAGTGGTTCCCGGATGAA | SEQ ID 96 - PSI_BW_5_G_allele: 5Hex-AGCCACGAAATG-3IABkFQ<br>SEQ ID 97 - PSI_BW_5_A_allele: 56FAM- AGCCACAAAATGA-3IABkFQ |
| SEQ ID 98 - PSI_BW_6 | SEQ ID 17 - PSI-BW6: AAGCA[G/T]CATCAA | Berry weight:<br>T = increased berry weight<br>G = decreased berry weight | SEQ ID 99 - PSI_BW_6_FOR: TGAAATGTGATAGTTGGTAGGTAAAG<br>SEQ ID 100 - PSI_BW_6_REV: CACCGCTTGGCTTTCTATTG | SEQ ID 101 - PSI_BW_6_G_allele: 5Hex- AAGCAGCATCAA-3IABkFQ<br>SEQ ID 102 - PSI_BW_6_T_allele: 56FAM- AAGCATCATCAACG-3IABkFQ |

**FIG. 4D**

## Macrophomina CIPHER™ Validation Results

| variety | 252_Id | Mac_AUDPC | COLMAC4 | COLMAC2 | COLMAC3 | FUS1 | FUS2 | Allele Score |
|---|---|---|---|---|---|---|---|---|
| bg 2.4306 | bg_2_4306_ntc | 1.2 | 0 | 1 | 0 | 0 | 0 | -1 |
| bg 10.3169 | bg_10_3169_ntc | 1.2 | 1 | 0 | 0 | 0 | 0 | 1 |
| bg 13.3305 | bg_2013_350_065 | 1.2 | 0 | 1 | 0 | 0 | 0 | -1 |
| bg 602.046 | 602_046 | 1.3 | 0 | 0 | 0 | 0 | 0 | 0 |
| bg 6.3016 | bg_6_3016_ntc | 1.4 | 0 | 1 | 0 | 0 | 0 | -1 |
| bg 367.038 | 367_038 | 1.6 | 0 | 1 | 0 | 0 | 0 | -1 |
| bg 10.3165 | bg_10_3165_ntc | 1.7 | 0 | 0 | 0 | 0 | 0 | 0 |
| bg 644.019 | 644_019 | 1.7 | 0 | 1 | 0 | 0 | 0 | -1 |
| bg 14.380.005 | 380_005 | 2.5 | 0 | 1 | 0 | 0 | 0 | -1 |
| bg 10.3194 | bg_10_3194_ntc | 2.6 | 0 | 1 | 0 | 0 | 0 | -1 |
| bg 8.3105 | bg_8_3105_ntc | 2.9 | 0 | 1 | 0 | 0 | 0 | -1 |
| bg 13.3297 | bg_2013_340_068 | 3.1 | 0 | 1 | 0 | 0 | 0 | -1 |
| UC Cabrillo | Cabrillo | 3.7 | 0 | 1 | 0 | 0 | 0 | -1 |
| sb 106.071 | sb_2013_106_071 | 3.8 | 0 | 1 | 0 | 0 | 0 | -1 |
| bg 404.066 | 404_066 | 4 | 0 | 1 | 0 | 0 | 0 | -1 |
| pep 818.101 | 818_101 | 4 | 0 | 1 | 0 | 0 | 0 | -1 |
| pep 834.069 | 834_069 | 4.2 | 0 | 1 | 0 | 0 | 0 | -1 |
| pe 183.023 | pe_2013_183_023 | 4.3 | 0 | 1 | 0 | 0 | 0 | -1 |
| pep 857.066 | 857_066 | 4.3 | 0 | 1 | 0 | 0 | 0 | -1 |
| bg 3.219.068 | bg_2003_219_068 | 5.3 | 0 | 0 | 0 | 0 | 0 | 0 |
| bg 10.3168 | bg_10_3168_ntc | 5.4 | 1 | 0 | 0 | 0 | 0 | 1 |
| pc 9271 | pc_0_9271_ntc | 5.4 | 0 | 1 | 0 | 0 | 0 | -1 |
| bg 2.4316 | bg_2_4316_ntc | 5.4 | 1 | 0 | 1 | 0 | 0 | 2 |
| pe 248.001 | pe_2014_248_001 | 5.5 | 0 | 1 | 0 | 0 | 0 | -1 |
| pe 4.152 | pe_4_152 | 5.5 | 0 | 1 | 0 | 0 | 0 | -1 |
| sb 205.006 | sb_2014_205_006 | 5.6 | 0 | 1 | 0 | 0 | 0 | -1 |
| pc 97.5298 | pc_97_5298_ntc | 5.6 | 0 | 1 | 0 | 0 | 0 | -1 |
| sb 273.029 | sb_2015_273_029 | 5.7 | 0 | 1 | 0 | 0 | 0 | -1 |
| sb 213.030 | sb_2015_213_030 | 6 | 1 | 0 | 1 | 1 | 0.5 | 3.5 |
| pc 89.1031 | pc_89_1031_tc | 6.1 | 0 | 1 | 0 | 1 | 0 | 0 |
| bg 4.352 | bg_4_352_ntc | 6.7 | 1 | 0 | 1 | 1 | 0.5 | 3.5 |
| pc 630.001 | 630_001 | 6.8 | 0 | 1 | 0 | 1 | 0 | 0 |
| pep 12.6003 | PEP_12_6003 | 6.8 | 0 | 1 | 0 | 1 | 0 | 0 |
| bg 6.3010 | bg_6_3010_ntc | 6.8 | 0 | 1 | 0 | 0 | 0 | -1 |
| sb 164.042 | sb_2013_164_042 | 6.8 | 0 | 1 | 0 | 1 | 0 | 0 |
| bg 95.633 | bg_95_633_ntc | 7 | 1 | 1 | 1 | 1 | 0.5 | 2.5 |
| sb 225.001 | sb_2014_225_001 | 7.2 | 0 | 1 | 0 | 0 | 0 | -1 |
| sb 279.024 | sb_2014_279_024 | 7.3 | 0 | 1 | 0 | 0 | 1 | 0 |
| bg 606.014 | 606_014 | 7.6 | 1 | 0 | 1 | 1 | 1 | 4 |
| UC Portola | PORTOLA | 7.6 | 1 | 1 | 1 | 0.5 | 1 | 2.5 |
| pep 853.035 | 853_035 | 7.8 | 0 | 1 | 0 | 0 | 0 | -1 |
| sb 302.047 | sb_2015_302_047 | 8.1 | 0 | 1 | 0 | 0 | 0 | -1 |
| UC Fronteras | FRONTERAS | 8.1 | 1 | 0 | 1 | 1 | 0.5 | 3.5 |
| sb 89.060 | sb_2015_89_060 | 8.2 | 0 | 1 | 0 | 0 | 0 | -1 |
| UC Albion | ALBION | 8.2 | 1 | 0 | 1 | 1 | 0.5 | 3.5 |
| UC San Andreas | SANANDREAS | 8.2 | 1 | 0 | 1 | 1 | 0.5 | 3.5 |
| bg 12.3250 | bg_2012_241_046 | 8.3 | 0 | 1 | 0 | 0 | 0 | -1 |
| pc 8.1120 | pc_8_1120_ntc | 8.3 | 0 | 1 | 0 | 0 | 0 | -1 |
| pc 86.118 | pc_86_118_tc | 8.6 | 1 | 0 | 1 | 1 | 1 | 4 |
| pe 261.068 | pe_2012_261_068 | 8.7 | 0 | 1 | 0 | 0 | 0 | -1 |
| sb 263.055 | sb_2015_263_055 | 8.8 | 1 | 0 | 1 | 1 | 0.5 | 3.5 |
| bg 12.3270 | bg_2012_367_010 | 9.2 | 1 | 0 | 1 | 1 | 0.5 | 3.5 |
| bg 13.3255 | bg_2012_259_063 | 9.3 | 0 | 1 | 0 | 0 | 0 | -1 |
| sb 61.041 | sb_2015_61_041 | 9.3 | 0 | 1 | 0 | 0 | 0 | -1 |
| sb 223.041 | sb_2014_223_041 | 9.3 | 0 | 0 | 0 | 0 | 0 | 0 |
| sb 430.066 | sb_2015_430_066 | 9.4 | 1 | 0 | 1 | 1 | 0.5 | 3.5 |
| sb 305.038 | sb_2014_305_038 | 9.4 | 0 | 1 | 0 | 0 | 1 | 0 |
| pe 143.004 | pe_2014_143_004 | 9.4 | 0 | 1 | 0 | 0 | 0 | -1 |
| sb 155.018 | sb_2013_155_018 | 9.5 | 1 | 0 | 1 | 1 | 0.5 | 3.5 |
| sb 68.089 | sb_2012_68_089 | 9.6 | 0 | 1 | 0 | 0 | 0 | -1 |
| pe 229.031 | pe_2014_229_031 | 9.6 | 0 | 1 | 0 | 0 | 0 | -1 |
| pe 11.2162 | pe_11_2162_ntc | 9.6 | 0 | 1 | 0 | 0 | 0 | -1 |
| UC Monterey | MONTEREY | 9.6 | 0 | 1 | 1 | 1 | 0 | 1 |
| sb 224.052 | sb_2014_224_052 | 9.6 | 1 | 0 | 1 | 1 | 0.5 | 3.5 |
| pep 500.032 | PEP_500_032 | 9.6 | 0 | 1 | 0 | 0 | 0 | -1 |
| pe 119.024 | pe_2013_119_024 | 9.6 | 1 | 0 | 0 | 1 | 1 | 3 |
| pep 458.059 | PEP_458_059 | 9.6 | 0 | 1 | 0 | 1 | 0 | 0 |
| sb 382.044 | sb_2015_382_044 | 9.6 | 0 | 0 | 1 | 1 | 0 | 2 |

82% Effective at Identifying the Susceptible with Three Errors in the Intermediate Zone. 90% Effective at Identifying Highly Susceptible.

63% Effective at Identifying the Resistant, However Macrophomina is Difficult to Screen Due to Escapes so it is Likely the Percent Effectiveness is Much Higher.

FIG. 5A

## Colletotrichum CIPHER™ Validation Results

| variety | 252_Id | Col_AUDPC | Allele Score |
|---|---|---|---|
| bg 2.4306 | bg_2_4306_rec | 1 | 1 |
| bg 6.3016 | bg_6_3016_rec | 1 | 2 |
| bg 367.038 | 367_038 | 1 | 1 |
| bg 10.3165 | bg_10_3165_rec | 1 | 4 |
| bg 14 380.005 | 380_005 | 1 | 1 |
| sb 186.071 | sb_2013_186_071 | 1 | 1 |
| pe 183.023 | pe_2013_183_023 | 1 | 1 |
| pe 248.001 | pe_2014_248_001 | 1 | 1 |
| sb 273.029 | sb_2015_273_029 | 1 | 1 |
| ps 630.001 | 630_001 | 1 | 1 |
| sb 225.001 | sb_2014_225_001 | 1 | 1 |
| bg 606.014 | 606_014 | 1 | 4 |
| sb 302.047 | sb_2015_302_047 | 1 | 1 |
| bg 12.3230 | bg_2012_241_046 | 1 | 1 |
| ps 86.118 | ps_86_118_tc | 1 | 4 |
| sb 263.055 | sb_2015_263_055 | 1 | 4 |
| sb 430.066 | sb_2015_430_066 | 1 | 4 |
| sb 305.038 | sb_2014_305_038 | 1 | 2 |
| sb 68.086 | sb_2012_68_086 | 1 | 1 |
| bg 602.046 | 602_046 | 1.1 | 4 |
| pep 857.066 | 857_066 | 1.1 | 1 |
| pep 12.0003 | PEP_12_0003 | 1.1 | 1 |
| sb 88.060 | sb_2015_88_060 | 1.1 | 1 |
| pe 229.031 | pe_2014_229_031 | 1.1 | 1 |
| pe 11.2162 | pe_11_2162_rec | 1.1 | 1 |
| UC Cabrillo | Cabrillo | 1.2 | 1 |
| bg 404.066 | 404_066 | 1.2 | 1 |
| sb 213.030 | sb_2015_213_030 | 1.5 | 5 |
| bg 6.3010 | bg_6_3010_rec | 1.7 | 1 |
| UC Portola | PORTOLA | 1.7 | 3 |
| ps 8.1120 | ps_8_1120_rec | 1.7 | 1 |
| bg 10.3194 | bg_10_3194_rec | 1.9 | 1 |
| bg 10.3169 | bg_10_3169_rec | 2 | 5 |
| bg 13.3305 | bg_2013_300_065 | 2 | 1 |
| sb 205.006 | sb_2014_305_006 | 2 | 1 |
| ps 97.5298 | ps_97_5298_rec | 2 | 2 |
| ps 89.1031 | ps_89_1031_tc | 2 | 1 |
| UC Fronteras | FRONTERAS | 2 | 5 |
| pe 143.056 | pe_2014_143_056 | 2 | 1 |
| sb 155.018 | sb_2013_155_018 | 2 | 5 |
| ps 9271 | ps_0_9271_rec | 3 | 2 |
| bg 2.4316 | bg_2_4316_rec | 3 | 6 |
| UC Albion | ALBION | 3 | 9 |
| sb 224.052 | sb_2016_224_052 | 3 | 6 |
| bg 12.3270 | bg_2012_367_010 | 3.5 | 6 |
| bg 4.352 | bg_4_352_rec | 3.7 | 6 |
| ps 4.152 | ps_4_152 | 4 | 3 |
| bg 12.3250 | bg_2012_258_093 | 4.8 | 3 |
| UC San Andreas | SANANDREAS | 5 | 6 |
| pe 261.068 | pe_2012_261_068 | 5 | 3 |
| psp 834.069 | 834_069 | 5.1 | 3 |
| bg 8.3105 | bg_8_3105_rec | 5.6 | 3 |
| pep 500.032 | PEP_500_032 | 5.8 | 3 |
| sb 61.041 | sb_2015_61_041 | 6 | 3 |
| pe 119.024 | pe_2013_119_024 | 6 | 7 |
| bg 644.019 | 644_019 | 6.1 | 3 |
| pep 858.101 | 858_101 | 6.1 | 3 |
| sb 223.041 | sb_2014_223_041 | 6.1 | 4 |
| bg 95.633 | bg_95_633_rec | 6.9 | 6 |
| pep 458.059 | PEP_458_059 | 7 | 3 |
| sb 382.044 | sb_2015_382_044 | 8.3 | 7 |

73% Effective at Identifying the Susceptible.

95% Effective at Identifying the Resistant.

FIG. 5B

## Berry Weight CIPHER$^{TM}$ Quantitative and Qualitative Models

### Berry Weight CIPHER (Complete)

| Marker | Ch (Vesa) | Position (Mb) | Estimate | NegLog10 | Effect (28.701) |
|---|---|---|---|---|---|
| BW_2 | 2 | 28437740 | NA | NA | 0.801 |
| BW_3_1 | 3 | 3055490 | 0.83 | 10.29 | |
| BW_3_2 | 3 | 7261822 | -2.57 | 27.13 | -1.115 |
| BW_3_3 | 3 | 7261835 | -2.57 | 27.13 | -1.115 |
| BW_4_1 | 4 | 27271385 | -0.98 | 11.23 | |
| BW_4_2 | 4 | 30025293 | -2.13 | 10.38 | -2.698 |
| BW_4_3 | 4 | 31410532 | -2.54 | 23.78 | -2.038 |
| BW_5 | 5 | 29308412 | 1.69 | 14.98 | 1.834 |
| BW_6 | 6 | 4244971 | -1.74 | 8.84 | |

### Berry Weight CIPHER (Adjusted)

<table>
<tr><th>Result</th><th>BW_2</th><th>BW_5</th><th>BW_3_2</th><th>BW_4_2</th></tr>
<tr><td><28.7</td><td colspan="2">0</td><td colspan="2">0/1</td></tr>
<tr><td><28.7</td><td colspan="2">0</td><td colspan="2">1</td></tr>
<tr><td><28.7</td><td colspan="2">1</td><td colspan="2">1</td></tr>
<tr><td>=28.7</td><td colspan="2">0</td><td colspan="2">0</td></tr>
<tr><td>=28.7</td><td colspan="2">1</td><td colspan="2">0/1</td></tr>
<tr><td>>28.7</td><td colspan="2">1</td><td colspan="2">0</td></tr>
</table>

FIG. 6

Europäisches Patentamt
European Patent Office
Office européen des brevets

# EUROPEAN SEARCH REPORT

Application Number
EP 22 16 9313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PINCOT DOMINIQUE D A ET AL: "Genome-Wide Association Mapping Uncovers Fw1 , a Dominant Gene Conferring Resistance to Fusarium Wilt in Strawberry", G3 GENES¦GENOMES¦GENETICS, vol. 8, no. 5, 1 May 2018 (2018-05-01), pages 1817-1828, XP055957065, DOI: 10.1534/g3.118.200129 Retrieved from the Internet: URL:http://academic.oup.com/g3journal/article-pdf/8/5/1817/37126192/g3journal1817.pdf> * page 1819, "DNA Marker Genotyping" * * the whole document * ----- | 1-9 | INV. C12Q1/6895 |
| Y | NAHLA V BASSIL ET AL: "Development and preliminary evaluation of a 90 K Axiom® SNP array for the allo-octoploid cultivated strawberry Fragaria x ananassa", BMC GENOMICS, vol. 16, no. 1, 7 March 2015 (2015-03-07), XP055556018, DOI: 10.1186/s12864-015-1310-1 * page 9, "Genotyping in octoploid strawberry" * * the whole document * ----- -/-- | 1-9 | TECHNICAL FIELDS SEARCHED (IPC) C12Q |

The present search report has been drawn up for all claims

| Place of search | Date of completion of the search | Examiner |
|---|---|---|
| Munich | 6 September 2022 | Marchesini, Patrizia |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 22 16 9313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SOORIYAPATHIRANA S D S S ET AL: "Using SNP/INDEL diversity patterns to identify a core group of genotypes from FVC11, a superior hybrid family ofMiller and(L.) Miller", GENETIC RESOURCES AND CROP EVOLUTION, KLUWER, DORDRECHT, NL, vol. 66, no. 8, 15 September 2019 (2019-09-15), pages 1691-1698, XP036923559, ISSN: 0925-9864, DOI: 10.1007/S10722-019-00819-0 [retrieved on 2019-09-15] * the whole document * ----- | 1-9 | |
| A | BERRY GENETICS INC: "Fragaria x ananassa Duchesne ex Rozier ; bg-11.3205", COMMUNITY PLANT VARIETY OFFICE, CPVO, 3 BOULEVARD MARÉCHAL FOCH CS 10121 49101 ANGERS CEDEX 2 - FRANCE, 26 December 2019 (2019-12-26), XP090008792, [retrieved on 2020-02-03] * the whole document * ----- | 1-9 | TECHNICAL FIELDS SEARCHED (IPC) |

The present search report has been drawn up for all claims

2

| Place of search | Date of completion of the search | Examiner |
|---|---|---|
| Munich | 6 September 2022 | Marchesini, Patrizia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2